(19) Europäisches Patentamt
European Patent Office
Office européen des brevets

(11) EP 3 977 863 A1

# (12) EUROPEAN PATENT APPLICATION

(43) Date of publication:
**06.04.2022 Bulletin 2022/14**

(21) Application number: **20199475.3**

(22) Date of filing: **30.09.2020**

(51) International Patent Classification (IPC):
***A23K 10/14*** *(2016.01)* ***A23K 10/30*** *(2016.01)*
***A23L 5/20*** *(2016.01)* ***A23L 7/104*** *(2016.01)*
***C12P 17/18*** *(2006.01)*

(52) Cooperative Patent Classification (CPC):
**A23K 50/30; A23K 10/14; A23K 10/30; A23K 20/147; A23L 5/25; A23L 7/107; C12P 17/181; C12Y 101/01002; C12Y 206/01**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Erber Aktiengesellschaft**
**3131 Getzersdorf bei Traismauer (AT)**

(72) Inventors:
- **Binder, Eva Maria**
  **3430 Tulln (AT)**
- **Streit, Elisabeth**
  **1220 Wien (AT)**
- **Brader, Günther**
  **1230 Wien (AT)**
- **Bernard, Claudia**
  **3438 Grafenwörth (AT)**
- **Weber, Barbara**
  **1150 Wien (AT)**
- **Lia, Martinez Montero**
  **4027 Debrecen (HU)**
- **Schrittwieser, Jörg**
  **8010 Graz (AT)**
- **Kroutil, Wolfgang**
  **8042 Graz (AT)**
- **Milica, Pastar**
  **1230 Wien (AT)**
- **Sessitsch, Angela**
  **1140 Wien (AT)**
- **Dolinsek, Jan**
  **1210 Wien (AT)**

(74) Representative: **Schiweck Weinzierl Koch Patentanwälte Partnerschaft mbB**
**Ganghoferstraße 68 B**
**80339 München (DE)**

Remarks:
Amended claims in accordance with Rule 137(2) EPC.

## (54) MEANS AND METHODS TO DETOXIFY MYCOTOXINS

(57) The present invention relates to polypeptides capable of modifying the C8-atom of deoxynivalenol and methods (e.g., for detoxifying mycotoxins) based thereon. The present invention further relates to compositions, kits, transgenic plants, transgenic seeds, transgenic pollen grains, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement, prebiotic, intermediate prebiotic and/or mixture/s thereof comprising one or more of the polypeptides capable of modifying the C8-atom of deoxynivalenol.

EP 3 977 863 A1

## Description

This application contains a Sequence Listing in a computer readable form, which is incorporated herein by reference.

## Technical field

The present invention relates to polypeptides capable of modifying the C8-atom of deoxynivalenol (DON) and/or DON derivative/s and methods (e.g., for detoxifying mycotoxins) based thereon. The present invention further relates to compositions, kits, transgenic plants, transgenic seeds, transgenic pollen grains, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement, prebiotic, intermediate prebiotic and/or mixture/s thereof comprising one or more of the polypeptides capable of modifying the C8-atom of deoxynivalenol and/or DON derivative/s. The present invention further relates to various suitable transgenic (e.g., recombinant) production host/s (e.g., bacterial cell, yeast cell, mammalian cell, insect cell and/or plant cell) and methods for bio-transformation of trichothecenes (e.g., DON or DON-derivative) to a molecule less toxic than the original corresponding trichothecene molecule (e.g., DON or DON-derivative), e.g., using the polypeptides of the present invention for said bio-transformation (e.g., wherein a hydroxyl group (-OH) at the C7-atom of the carbon chain of the corresponding trichothecene compound (e.g., DON or DON-derivative) remains unchanged during said enzymatic bio-transformation).

## Background of the invention

Mycotoxins are secondary metabolites of filamentous fungi. These fungi grow inter alia on various types of grain and cereals. Typically, the crops are infested by the fungi prior to harvest and mycotoxin production can occur before and after harvest as well as during storage. According to the Food and Agriculture Organization, approximately 25% of all agricultural products are contaminated with mycotoxins causing considerable economic losses. Among 19,757 samples analyzed from January 2004 to December 2011, 72% of the samples tested positive for at least one mycotoxin, and 39% were co-contaminated (Schatzmayr and Streit. 2013. World Mycotoxin Journal 6(3): 213-222). Mycotoxins are known to cause serious adverse health effects in humans and animals, including mutagenic, cancerogenic, neurotoxic, immunosuppressive or teratogenic effects. A particularly large group of mycotoxins are trichothecene mycotoxins or trichothecenes. Trichothecenes are a class of sesquiterpenes which are produced *inter alia* by *Fusarium, Myrothecium, Podostroma, Trichoderma* or *Trichothecium* species and may comprise an 12,13-epoxy ring (that may interchangeably be referred to herein as "12,13-epoxytrichothec-9-ene moiety" or "trichothecene-ring"), as a common structural feature:

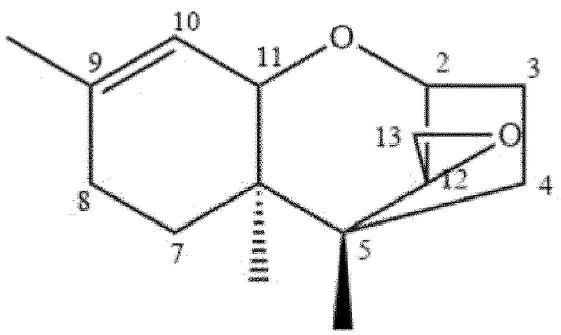

The family of trichothecene mycotoxins comprises deoxynivalenol (DON, CAS no. 51481-10-8), T-2 toxin (CAS no. 21259-20-1), HT-2 toxin (CAS no. 26934-87-2), nivalenol (CAS no. 23282-20-4), fuseranon-X (CAS no. 23255-69-8), scripentriol, 15-acetoxyscirpenol (CAS no. 2623-22-5), 4,15-diacetoxyscirpenol (CAS no. 2270-40-8), trichodermol (CAS no. 2198-93-8), verrucarin A (CAS no. 3148-09-2), verrucarin J (CAS no. 4643-58-7), isotrichodermin (CAS no. 91423-90-4), hydroxyisotrichodermin (CAS no. 344781-02-8), calonectrin (CAS no. 38818-51-8), T-2 tetraol (CAS no. 34114-99-3), deacetylneosolaniol (CAS no. 74833-39-9), neosolaniol (CAS no. 36519-25-2), acetylneosolaniol (CAS no. 65041-92-1), sporotrichiol (CAS no. 101401-89-2), trichotriol (CAS no. 109890-37-1), sambucinol (CAS no. 90044-33-0), and culmorin (CAS no. 18374-83-9).

Type B trichothecenes as referred herein may refer to trichothecene compounds having a carbonyl group (C=O) at the position 8 of the carbon chain (or, in other words, an oxygen atom (=O), doubly bonded to the C8-atom of the carbon chain) and may include DON-derivatives. DON-derivatives as referred herein may refer to trichothecene compounds having a carbonyl group (C=O) at the position 8 of the carbon chain (or, in other words, an oxygen atom (=O), doubly bonded to the C8-atom of the carbon chain) and a hydroxyl group (-OH) at the C7-atom of the carbon chain. Exemplary DON-derivatives (or Type B trichothecenes) as referred herein may include:

| Name | Abbreviation | Chemical | Formula | MW | CAS no |
|---|---|---|---|---|---|
| Deoxynivale nol | DON | (3α, 7α)-3,7,15-trihydroxy-12,13-epoxytrichothec-9-en-8-one | $C_{15}H_{20}O_6$ | 296.3 | 51481-10-8 |
| 3-Acetyldeoxy nivalenol | 3A-DON | 3Acetyloxy-3α,7α-dihydroxy-12,13-epoxytrichothec-9-en-8-one | $C_{17}H_{22}O_7$ | 338.4 | 50722-38-8 |
| 3-keto Deoxynivale nol | 3K-DON | (7α)-7,15-dihydroxy-12,13-epoxytrichothec-9-en-3,8-one | $C_{15}H_{18}O_6$ | 294.3 | Not available |
| 3-epi Deoxynivale nol | 3E-DON | (3β, 7α)-3,7,15-trihydroxy-12,13-epoxytrichothec-9-en-8-one | $C_{15}H_{20}O_6$ | 296.3 | Not available |
| 15-Acetyldeoxy nivalenol | 15A-DON | 15-Acetyloxy-3α,7α-dihydroxy-12,13-epoxytrichothec-9-en-8-one | $C_{17}H_{22}O_7$ | 338.4 | 88337-96-6 |
| Nivalenol | NIV | (3α,4β,7α)-3,4,7,15-tetrahydroxy-12,13-epoxytrichothec-9-en-8-one | $C_{15}H_{20}O_7$ | 312.3 | 23282-20-4 |
| Fusarenon-X (4-acetylnivale nol) | FusX | (3β, 4α,7α)-3,7,15-trihydroxy-8-oxo-12,13-epoxytrichothec-9-en-4-yl acetate | $C_{17}H_{22}O_8$ | 354.4 | 23255-69-8 |
| 3-amino Deoxynivale nol | 3-amino DON | 3-amino-(7α)-7,15-dihydroxy-12,13-epoxytrichothec-9-en-8-one | $C_{15}H_{21}NO_5$ | 295.3 | Not available |

In animals, the consumption of trichothecene mycotoxins leads to reduced feed intake, impaired nutrient absorption and reduced growth, vomiting, diarrhea, immunological dysfunctions and reduced milk production. In humans, adverse health effects include nausea, vomiting, diarrhea, abdominal pains, headaches and fever. Especially crop contamination with DON (IUPAC name (3α,7α)-3,7,15-trihydroxy-12,13-epoxytrichothec-9-en-8-one) can be found all around the world and several additional toxic DON derivatives have been described, *e.g.* acetylated DON (such as 15-acetyl-DON, 15-ADON), glycosylated DON, DON sulfonate (such as DONS-1 or DONS-2), or DON sulfate (such as DON-15-sulfate). National and regional authorities have therefore established maximum levels for DON in food (EC no. 1881/2006, EC no. 1126/2007) or feed (2006/576/EC) to avoid mycotoxicosis of humans and animals upon DON ingestion, leading to undesirable effects such as the inhibition of protein biosynthesis, interactions with serotonin and dopamine receptors or upregulation of pro-inflammatory cytokines (EFSA Journal 2004, 73, 1-41).

The risk of contamination with some mycotoxins can be reduced by applying "good agricultural practice". Despite such precautions, DON contamination cannot be avoided and additional strategies are necessary to avoid DON mycotoxicosis. A common strategy to combat mycotoxicosis is the addition of binding agents such as clays, yeast or yeast products to the feed. However, due to its hydrophilic character, DON does not interact with commonly employed binding agents and thus cannot be removed by such a strategy. Similarly, DON cannot be removed from food or feed by chemical or physical treatment.

As an alternative strategy to avoid mycotoxicosis, DON could be detoxified by transformation to a less toxic molecule.

EP 1 042 449 A1 or US 2012/0263827 A1 relate to microorganisms capable of detoxifying trichothecenes by cleaving of the epoxy ring on the C12 and C13 atoms or by biotransformation to 3-epi-DON and 3-keto-DON, respectively. DON detoxification strategies using microorganisms based on the C3 atom are known in the art. Generally, the use of such microorganisms is often challenged by the specific cultivation requirements of these microorganisms, rendering their production economically unfavorable. In addition, the admixture of microorganisms to feed or food is not feasible in many food or feed processes. Therefore, biotransformation of DON by a microorganism-free enzyme formulation is needed as fast and safe means for DON detoxification. In this regard, WO 2016/154640 A1 describes an alcohol dehydrogenase containing metal ions and a quinone cofactor for transforming a trichothecene having a hydroxyl-group at the C3 atom. CN 107916266 A describes a multi-enzyme process for the biotransformation of DON to 3-epi-DON. In 2012, a three-enzyme system comprising the cytochrome P450 enzyme DdnA derived from *Sphingomonas sp.* KSM1 was described to perform a hydroxylation of DON to 16-hydroxy-DON (Ito et al. 2012. Appl Environ Microbiol 79(5):1619-1628).

Similarly, another *Sphingomonas* strain, S3-4, was described to transform DON to 3-oxo-DON and 3-epi-DON (He et al. 2017. Scientific Reports. 27:9549). In the course of the study, the aldo-keto reductase Akr18A1 from strain S3-4 was described to be capable of transforming DON to 3-oxo-DON, albeit in a reversible redox reaction.

Enzymes of the aldo-keto reductase protein family share a common protein fold, an $(\alpha/\beta)_8$-barrel, also called a TIM barrel structure, and a catalytic tetrad consisting of the amino acids aspartic acid, tyrosine, lysine and histidine (Mindnich and Penning. 2009. Hum Genomics 3(4): 362-370). Also, the PQQ-dependent dehydrogenase DepA from *Devosia mutans* 17-2-E-8 was described to transform DON to 3-keto-DON (Carere et al. 2017. Microb Biotechnol, DOI: 10.1111/1751-7915.12874).

The present invention was made in view of the prior art outlined above. The objective of the present invention can be *inter alia* formulated as to provide means and methods to detoxify deoxynivalenol (DON and/or DON-derivatives) to a molecule less toxic than DON, which molecule is not transformed back to DON. The solution of the present invention is described in the following, exemplified in the examples, illustrated in the figures and reflected in the claims.

This objective has been achieved by providing a method for a biotransformation of DON to a molecule less toxic than DON, comprising the steps of providing a polypeptide, contacting the polypeptide with DON and an aqueous liquid, and performing a biochemical reaction on DON, wherein the biochemical reaction is a modification of the C8 atom of DON.

DON is a trichothecene mycotoxin having a carbonyl group (C=O) at the position 8 of the carbon chain (or, in other words, an oxygen atom (=O), doubly bonded to the C8-atom of the carbon chain) and a hydroxyl group (-OH) at the C7-atom of the carbon chain with the following structural formula, wherein the C8 atom is indicated:

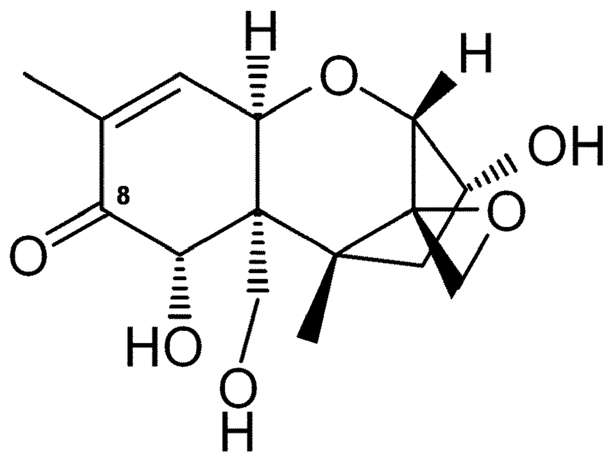

**Summary of the invention**

The present invention relates to a polypeptide capable of modifying the C8-atom of a Type B trichothecene, preferably DON or derivative thereof, wherein said polypeptide is selected from the group consisting of: (i) a polypeptide having at least 70% sequence identity (e.g., at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100%) to the polypeptide of: SEQ ID NO: 1 (e.g., "Akr I"), SEQ ID NO: 2 (e.g., "Akr II"), SEQ ID NO: 3 (e.g., "Akr III"), SEQ ID NO: 4 (e.g., "ADH-Lk") or SEQ ID NO: 5 (e.g., "TAM-Ac"); (ii) a variant of the polypeptide of SEQ ID NO: 1, SEQ ID NO: 2, SEQ ID NO: 3, SEQ ID NO: 4 or SEQ ID NO: 5, wherein said variant comprising a substitution, deletion, and/or insertion at one or more positions; (iii) a fragment of the polypeptide of (a) or (b) capable of modifying the C8-atom of DON and/or a DON derivative/s.

The present invention further relates to a method for one or more of the following (e.g., in/for a substrate or sample, e.g., seed/s): (i) for modifying the C8-atom of DON; (ii) for altering the level of DON (e.g., reducing DON level) and/or detoxifying DON; (iii) for bio-transforming (e.g., enzymatically transforming, processing and/or converting) DON, preferably said bio-transforming is enzymatic; (iv) for detecting and/or assessing the level of DON; and/or (v) for inhibiting toxicity of DON and/or for treating seeds; said method comprising: (a) providing one or more of the following: polypeptide/s, compound/s, intermediate/s, polynucleotide/s, nucleic acid construct/s, expression vector/s, recombinant host cell/s, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic

or mixture/s thereof, composition or kit according to the present invention to said substrate or sample; (b) applying (a) to a substrate or sample (e.g., seed/s, foodstuff, intermediate foodstuff; fodder, intermediate fodder; feed, intermediate feed); (c) preferably, detecting and/or quantifying one or more compound/s or intermediate/s according to the present invention.

The present application satisfies this demand by the provision of the variants described herein below, characterized in the claims and illustrated by the appended Examples and Figures.

**Overview of the sequence listing**

As described herein references can be made to UniProtKB Accession Numbers (http://www.uniprot.org/, e.g., as available in UniProtKB release 2020_03 Published June 17, 2020).

SEQ ID NO: 1 is the artificial amino acid sequence of the "Akr I" variant of a wild type aldo-keto reductase.
SEQ ID NO: 2 is the artificial amino acid sequence of the "Akr II" variant of a wild type aldo-keto reductase.
SEQ ID NO: 3 is the artificial amino acid sequence of the "Akr III" variant of a wild type aldo-keto reductase.
SEQ ID NO: 4 is the artificial amino acid sequence of the "ADH-Lk" variant of a wild type alcohol dehydrogenase.
SEQ ID NO: 5 is the artificial amino acid sequence of the "TAM-Ac" variant of a wild type transaminase.

**Brief description of the drawings**

**Figure 1:** Alignment of Akr I (SEQ ID NO: 1), Akr II (SEQ ID NO: 2) and Akr III (SEQ ID NO: 3) variants of the aldo-keto reductase.
**Figure 2:** Concentrations of DON and epi-THS in a DON transformation assay. The x-axis shows the time in min, the y-axis shows the concentrations of DON and epi-THS in ng/mL.
**Figure 3:** DON degradation activities of Akr I (SEQ ID NO: 1), Akr II (SEQ ID NO: 2) and Akr III (SEQ ID NO: 3) at different pH values. The respective maximum activities were set to 100%. The x-axis shows the pH value, the y-axis shows the relative DON degradation activities in %.
**Figure 4:** DON degradation activities of Akr I (SEQ ID NO: 1), Akr II (SEQ ID NO: 2) and Akr III (SEQ ID NO: 3) at different temperatures. The respective maximum activities were set to 100%. The x-axis shows the temperature in °C, the y-axis shows the relative DON degradation activities in %.
**Figure 5:** Non-linear curve fit to DON biotransformation data by Akr. The x-axis shows DON concentrations in $\mu$M, the y-axis shows the DON degradation rates in units per mg protein (U/mg), wherein one unit refers to the amount of enzyme that transforms one $\mu$mol of DON in one minute.
**Figure 6:** Metabolite toxicity of the compounds/intermediates of the present invention (e.g., as described in Example 7) measured as inhibition of translation of firefly luciferase in an *in vitro* transcription/translation assay. Observed maximum inhibition was set to 100%. The x-axis shows the concentrations of DON, epi-THS, and THS in $\mu$M, the y-axis shows the inhibition of translation in %.
**Figure 7:** Exemplary DON transformation reactions catalyzed by the variants of the present invention.

**Detailed description of the invention**

**Definitions**

As referred herein "EC numbers" (Enzyme Commission numbers) may be used to refer to enzymatic activity according to the Enzyme nomenclature database, Release of February 26, 2020 (e.g., available at https://enzyme.expasy.org/). The EC number refers to Enzyme Nomenclature 1992 from NC-IUBMB, Academic Press, San Diego, Calif., including supplements 1-5 published in Eur. J. Biochem. 1994, 223, 1-5; Eur. J. Biochem. 1995, 232, 1-6; Eur. J. Biochem. 1996, 237, 1-5; Eur. J. Biochem. 1997, 250, 1-6; and Eur. J. Biochem. 1999, 264, 610-650; respectively.

The term "polypeptide" is equally used herein with the term "protein". Proteins (including fragments thereof, preferably biologically active fragments, and peptides, usually having less than 30 amino acids) comprise one or more amino acids coupled to each other via a covalent peptide bond (resulting in a chain of amino acids). The term "polypeptide(s)" as used herein describes a group of molecules, which, for example, consist of more than 30 amino acids. Polypeptides may further form multimers such as dimers, trimers and higher oligomers, i.e. consisting of more than one polypeptide molecule. Polypeptide molecules forming such dimers, trimers etc. may be identical or non-identical. The corresponding higher order structures of such multimers are, consequently, termed homo- or heterodimers, homo- or heterotrimers etc. An example for a heteromultimer is an antibody molecule, which, in its naturally occurring form, consists

of two identical light polypeptide chains and two identical heavy polypeptide chains. The terms "polypeptide" and "protein" also refer to naturally modified polypeptides/proteins wherein the modification is affected e.g. by post-translational modifications like glycosylation, acetylation, phosphorylation and the like. Such modifications are well known in the art.

**Sequence identity:** The relatedness between two amino acid sequences or between two nucleotide sequences is described by the parameter "sequence identity". For purposes of the present invention, the sequence identity between two amino acid sequences is determined using the Needleman-Wunsch algorithm (Needleman and Wunsch, 1970, J. Mol. Biol. 48: 443-453) as implemented in the Needle program of the EMBOSS package (EMBOSS: The European Molecular Biology Open Software Suite, Rice et al., 2000, Trends Genet. 16: 276-277), preferably version 5.0.0 or later. The parameters used may be gap open penalty of 10, gap extension penalty of 0.5, and the EBLOSUM62 (EMBOSS version of BLOSUM62) substitution matrix. The output of Needle labeled "longest identity" (obtained using the no-brief option) is used as the percent identity and is calculated as follows:

$$(\text{Identical Residues} \times 100)/(\text{Length of Alignment} - \text{Total Number of Gaps in Alignment}).$$

Alternatively, the parameters used may be gap open penalty of 10, gap extension penalty of 0.5, and the EDNAFULL (EMBOSS version of NCBI NUC4.4) substitution matrix. The output of Needle labeled "longest identity" (obtained using the no-brief option) is used as the percent identity and is calculated as follows:

$$(\text{Identical Deoxyribonucleotides} \times 100)/(\text{Length of Alignment} - \text{Total Number of Gaps in Alignment}).$$

Expression: The term "expression" includes any step involved in the production of a variant (polypeptide) including, but not limited to, transcription, post-transcriptional modification, translation, post-translational modification, and secretion.

Expression vector: The term "expression vector" may refer to a linear or circular DNA molecule that comprises a polynucleotide encoding a variant (polypeptide) and is operably linked to control sequences that provide for its expression, in particular for its transcription.

Fragment: The term "fragment" may refer to a polypeptide having one or more (e.g. several) amino acids absent from the amino and/or carboxyl terminus of a mature polypeptide; wherein the fragment has an activity as described elsewhere herein.

Host cell: The term "host cell" may refer to any cell type that is susceptible to transformation, transfection, transduction, or the like with a nucleic acid construct or expression vector comprising a polynucleotide of the present invention. The term "host cell" encompasses any progeny of a parent cell that is not identical to the parent cell due to mutations that occur during replication.

Nucleic acid construct: The term "nucleic acid construct" may refer to a nucleic acid molecule, either single- or double-stranded, which is isolated from a naturally occurring gene or is modified to contain segments of nucleic acids in a manner that would not otherwise exist in nature or which is synthetic, which comprises one or more control sequences.

Operably linked: The term "operably linked" may refer to a configuration in which a control sequence is placed at an appropriate position relative to the coding sequence of a polynucleotide such that the control sequence directs expression of the coding sequence.

Control sequences: The term "control sequences" as used herein may refer to nucleic acid sequences necessary for expression of a polynucleotide encoding a variant (polynucleotide) of the present invention. Each control sequence may be native (i.e., from the same gene) or foreign (i.e., from a different gene) to the polynucleotide encoding the variant or native or foreign to each other. Such control sequences include, but are not limited to, a leader, polyadenylation sequence, pro-peptide sequence, promoter, signal peptide sequence, and transcription terminator. At a minimum, the control sequences include a promoter, and transcriptional and translational stop signals. The control sequences may be provided with linkers for the purpose of introducing specific restriction sites facilitating ligation of the control sequences with the coding region of the polynucleotide of the present invention.

As used herein, the term "corresponding to" may refer to a way of determining the specific amino acid of a sequence wherein reference is made to a specific amino acid sequence (e.g., US2020071638). E.g. for the purposes of the present invention, when references are made to specific amino acid positions, the skilled person would be able to align another amino acid sequence to said amino acid sequence that reference has been made to, in order to determine which specific amino acid may be of interest in said another amino acid sequence. Alignment of another amino acid sequence with e.g. the sequence as set forth in SEQ ID NOs: 1, 2, 3, 4 or 5 or any other sequence listed herein, has been described elsewhere herein. Alternative alignment methods may be used, and are well-known for the skilled person.

The term "position" when used in accordance with the present invention may refer to a position of an amino acid within an amino acid sequence depicted herein. The term "corresponding" in this context may include that a position is not only determined by the number of the preceding nucleotides/amino acids.

As used herein, "silent" mutations mean base substitutions within a nucleic acid sequence which do not change the amino acid sequence encoded by the nucleic acid sequence. "Conservative or equivalent" substitutions (or mutations) mean substitutions as listed as "Exemplary Substitutions" in Table I below. "Highly conservative" substitutions as used herein mean substitutions as shown under the heading "Preferred Substitutions" in Table I below.

TABLE I Amino Acid Substitutions

| Original | Exemplary Substitutions | Preferred Substitutions |
|---|---|---|
| Ala (A) | val; leu; ile | Val |
| Arg (R) | lys; gln; asn | lys |
| Asn (N) | gln; his; asp, lys; arg | gln |
| Asp (D) | glu; asn | glu |
| Cys (C) | ser; ala | ser |
| Gln (Q) | asn; glu | asn |
| Glu (E) | asp; gln | asp |
| Gly (G) | ala | ala |
| His (H) | asn; gln; lys; arg | arg |
| Ile (I) | leu; val; met; ala; phe; | leu |
| Leu (L) | norleucine; ile; val; met; ala; | ile |
| Lys (K) | arg; gln; asn | arg |
| Met (M) | leu; phe; ile | leu |
| Phe (F) | leu; val; ile; ala; tyr | tyr |
| Pro (P) | ala | ala |
| Ser (S) | thr | thr |
| Thr (T) | ser | ser |
| Trp (W) | tyr; phe | tyr |
| Tyr (Y) | trp; phe; thr; ser | Phe |
| Val (V) | ile; leu; met; phe; ala; | leu |

Variant: The term "variant" may refer to a polypeptide having specific activity as described herein comprising an alteration, i.e., a substitution, insertion, and/or deletion, at one or more (e.g., several) positions. A substitution means replacement of the amino acid occupying a position with a different amino acid; a deletion means removal of the amino acid occupying a position; and an insertion means adding an amino acid adjacent to and immediately following the amino acid occupying a position.

As used herein the term "transgenic" may refer to an organism whose genome has been altered by the incorporation of foreign genetic material or additional copies of native genetic material, e.g. by transformation or recombination (e.g., US7410800B2). The transgenic organism may be a plant, mammal, fungus, bacterium or virus. As used herein "transgenic plant, seed or pollen grain" may refer to a plant, seed or pollen grain or progeny plant, seed or pollen grain of any subsequent generation derived therefrom, wherein the DNA of the plant, seed or pollen grain or progeny thereof contains an introduced exogenous DNA not originally present in a non-transgenic plant, seed or pollen grain of the same strain. The transgenic plant, seed or pollen grain may additionally contain sequences which are native to the plant being transformed, but wherein the exogenous DNA has been altered in order to alter the level or pattern of expression of the coding sequence.

The term "foodstuff" may refer to a substance having a food value.

The term "fodder" may refer to a substance fed to domestic animals.

The term "feed" may refer to a substance used as food for livestock.

The term "additive" may refer to a compound or substance added to another product or substance, e.g., in a small amount, to affect a desired property and/or characteristics.

The term "prebiotic" may refer to a compound or substance capable of inducing the growth and/or activity of beneficial microorganisms.

The term "detoxifying agent" may refer to a compound or substance capable of reducing- and/or inhibiting toxicity.

The term "nutritional supplement" may refer to a compound or substance capable to support the nutritional content of the diet, e.g., vitamins and minerals.

The term "intermediate" may refer to a compound or substance produced during the process (e.g., during an intermediate stage of the process) of obtaining an end-product of the present invention, e.g., foodstuff, fodder, fodder; feed, additive (e.g., foodstuff-, fodder- or feed additive), detoxifying agent, nutritional supplement or prebiotic of the present invention.

It must be noted that as used herein, the singular forms "a", "an", and "the", include plural references unless the context clearly indicates otherwise. Thus, for example, reference to "a reagent" includes one or more of such different reagents and reference to "the method" includes reference to equivalent steps and methods known to those of ordinary skill in the art that could be modified or substituted for the methods described herein.

Unless otherwise indicated, the term "at least" preceding a series of elements is to be understood to refer to every element in the series. Those skilled in the art will recognize, or be able to ascertain using no more than routine experimentation, many equivalents to the specific embodiments of the invention described herein. Such equivalents are intended to be encompassed by the present invention.

The term "and/or" wherever used herein includes the meaning of "and", "or" and "all or any other combination of the elements connected by said term".

The term "about" or "approximately" as used herein means within 20%, preferably within 10%, and more preferably within 5% of a given value or range.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having".

When used herein "consisting of" excludes any element, step, or ingredient not specified in the claim element. When used herein, "consisting essentially of" does not exclude materials or steps that do not materially affect the basic and novel characteristics of the claim.
In each instance herein any of the terms "comprising", "consisting essentially of" and "consisting of" may be replaced with either of the other two terms.

The objective of the present invention has been achieved by providing means and methods as described herein, comprising the steps of providing one or more polypeptide/s of the present invention, contacting said one or more polypeptide/s with the Type B trichothecene of the present invention, preferably DON and/or DON-derivative/s, (e.g., in an aqueous phase) and performing a biochemical reaction on the Type B trichothecene of the present invention, preferably DON and/or DON-derivative/s, wherein the biochemical reaction is a modification of the C8-atom of the Type B trichothecene of the present invention, preferably DON and/or DON-derivative/s. Preferably wherein (i) said modifying is a transformation, e.g. substitution or the change of a functional group of a chemical species (e.g., carbonyl-, keto-, and/or oxo-moiety (=O) of the C8-atom of the Type B trichothecene of the present invention, preferably DON or DON derivative, into a different functional group (e.g., a hydroxyl (-OH) or amino (-NH2) moiety); and/or (ii) said modifying is modifying of only the C8-atom of the Type B trichothecene of the present invention, preferably DON or DON derivative/s; and/or (iii) said modifying consists of modifying the C8-atom of the Type B trichothecene of the present invention, preferably DON or DON derivative/s; and/or (iv) said modifying is not comprising molecular rearrangement, e.g., not comprising a change in which there is a bond migration of an atom or bond and/or an entering group takes up a different position from the leaving group, with accompanying bond migration; and/or (v) said modifying is not comprising modifying of the C7-atom of the Type B trichothecene of the present invention, preferably DON or DON derivative/s; and/or (vi) said modifying is not comprising isomerization (e.g., intramolecular isomerization) of the Type B trichothecene of the present invention, preferably DON or DON derivative/s, e.g., wherein the product of said modifying is isomeric with a corresponding reactant, e.g., the Type B trichothecene of the present invention, preferably DON or DON derivative.

By providing a method as described above, the inventors found that the molecule, which is the product of a biotransformation of the Type B trichothecene of the present invention, preferably DON, at its C8 atom, is less toxic than a corresponding original Type B trichothecene of the present invention, preferably DON, and that this product molecule is not transformed back to a corresponding original Type B trichothecene of the present invention, preferably DON in a reverse reaction. Hereby, the adverse health effects of the Type B trichothecene of the present invention, preferably DON, can be eliminated or at least reduced. By providing a method that leads to such a biotransformation product of the Type B trichothecene of the present invention, preferably DON, which product is not reverted back to a corresponding

original Type B trichothecene, preferably DON, the detoxification of the Type B trichothecene of the present invention, preferably DON, is lasting and reliable DON detoxification processes can be developed (e.g., Figure 7).

Biotransformation of DON to a molecule less toxic than DON at the C8 atom of DON was found to be performed especially well by providing means and methods as described herein, preferably wherein the polypeptide of the present invention (e.g., an Akr variant) is a cofactor-dependent oxidoreductase. Cofactors can be e.g. NADPH or NADH or other typical enzyme cofactors that are known to those skilled in the art.

In some aspects, concentrations of DON and epi-THS throughout the time course of a DON transformation assay carried out with Akr I-III variant (polypeptide) as described herein below, and exemplarily shown in Figure 2. By applying the DON transformation assay as described herein below in Example 3, e.g., at pH values ranging from pH 2 to pH 10, the pH optima for DON degradation of Akr I-III (SEQ ID NOs: 1-3) can be determined. Preferably, the Akr I and Akr II variants of the present invention can be activated at pH 6, whereas the Akr III variant at pH 6.5. For example, Akr I-III showed 50% activity or more of their respective maximum activity at pH values 4.5 and 5.0 up to pH 9.5 and pH 9.0. For the Akr I variant, at pH 4.0 the reaction proceeds slower, nevertheless the degradation DON can still be achieved, e.g., after 30 min. In other aspects, by applying the DON transformation assay of the present invention, e.g., as described in Example 3, but at different temperatures ranging from 14.9 °C to 55.7 °C, the temperature optima for the DON degradation activities of Akr I-III can be determined. The Akr I variant maximum activity is at approximately 35 °C, Akr II at approximately 31 °C, and Akr III at temperatures from approximately 28 °C to 33 °C (e.g., Figure 4). Akr I-III showed more than 50% of its maximum activity at temperatures from approximately 20 °C to 48 °C. For Akr I even at temperatures below 20 °C (like 14.9 °C), complete DON degradation could be achieved, e.g., after 20 min of incubation. In further aspects, exemplary relative DON-transformation activities of the Akr variants of the present invention during 4 h of storage at 30 °C at different pH values is provided in Tables 4-6 herein below. In some further aspects, exemplary relative DON transformation activities of the Akr variants during 60 min of incubation in storage buffer at different temperatures is provided in Tables 7-9 herein below.

In some aspects, the Akr I polypeptide (variant) of the present invention comprises one or more equivalent (or same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283, L284, particularly preferred D54, Y59, K84 and/or H125 position/s of SEQ ID NO: 1.

In some aspects, the Akr II polypeptide (variant) of the present invention comprises one or more equivalent (or same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283, L284, particularly preferred D54, Y59, K84 and/or H125 position/s of SEQ ID NO: 2.

In some aspects, the Akr III polypeptide (variant) of the present invention comprises one or more equivalent (or same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283, L284, particularly preferred D54, Y59, K84 and/or H125 position/s of SEQ ID NO: 3.

The efficiency of the biotransformation of DON as well as the quality of the reaction product can be influenced by the choice of the transformation catalyst. By applying a method as described above, wherein the Akr I-III and Akr variants of the present invention comprises an $(\alpha/\beta)_8$-barrel protein structure, it is possible to ensure the structural integrity of the biocatalyst, and hereby particularly good continuity of the biotransformation reaction. An $(\alpha/\beta)_8$-barrel protein structure, also referred to as TIM barrel, is a protein structure comprising eight $\alpha$-helices and eight $\beta$-sheets, as described e.g. by Mindnich and Penning (2009. Hum Genomics 3(4): 362-370). Herein, the terms polypeptide, protein or enzyme are used interchangeably.

In some aspects, the present invention further relates to DON transformation assay carried out with ADH-Lk variants (polypeptides) of the present invention (as described herein below, e.g., in Example 6, e.g., Figure 7). In particular, the present invention relates to reduction of DON to 3,7,8R,15-tetrahydroxyscirpine (THS) catalyzed by ADH-Lk variants of the present invention (e.g., SEQ ID NO. 4). In some aspects, DON conversion to THS with ADH-Lk variants of the present invention is carried out from 3.5 to 22 hours, e.g., at 30°C, e.g., Table 10 herein below. In preferred aspects, the reduction DON and/or its derivatives is catalyzed by ADH-Lk variants of the present invention to 3,7,8R,15-tetrahydroxyscirpine (THS). In some aspects, the ADH-Lk polypeptide (variant) of the present invention comprises one or more equivalent (or same) amino acid/s in position/s corresponding to: N114, S143, Y156 and/or K160 position/s of SEQ ID NO: 4; and/or G14, H17, G18, 119, G20, G38, R39, R40, H62, D63, V64, N90, A91, G92, I93, V113, M141, S142, S143, Y156, K160, P188, G189, P190, 1191, T193, P194 and/or L195 position/s of SEQ ID NO: 4; and/or E67, V99, E100, T102, T104, W107, R108, L111, L115, D116, F119, F120, T122, R123, 1126, Q127, K130, G146, Q147, G149, D150, P151, G154, S157, A158, G161, A162, R164, 1165, M166, K168, S169, A170, L172, L173 and/or C174 position/s of SEQ ID NO: 4; and/or R4, E67, V99, E100, T102, T104, W107, R108, L111, L115, D116, F119, F120, T122, R123, 1126, Q127, K130, G146, Q147, V148, G149, D150, P151, G154, S157, A158, G161, A162, R164, 1165, M166, K168, S169, A170, A171, L172, L173, C174, A175, P190, T212, P213, M214, G215, H216, 1217, G218, E219, D222, W225, V226, Y229, E234, K236, F237, A238, T239, G240, A241, E242, F243, V244, V245, D246, G247, G248, Y249, T250,

A251 and/or Q252 position/s of SEQ ID NO: 4. In some aspects, metabolites (e.g., compounds or intermediates of the present invention), epi-THS and THS are about 25-30 times less toxic in this assay compared to DON, e.g., Figure 6.

In some aspects, the present invention further relates to transamination of DON (e.g., Figure 7). Transaminases (EC 2.6.1.-) catalyze the reversible transfer of an amino group from an amine to a carbonyl compound (acceptor). In some aspects, transamination of DON and/or its derivatives with additional carbonyl groups, such as 3-keto DON to aminated metabolites (e.g., compounds or intermediates of the present invention, such as for example 8-amino-3-keto DON) catalyzed by, e.g., SEQ ID NO. 5 (TAM-Ac, as described herein below, e.g., in Example 8 herein below). In some aspects, the TAM-Ac polypeptide (variant) of the present invention comprises one or more equivalent (or same) amino acid/s in position/s corresponding to: K188, Y67, R86, K188, E221, I246, T247, T283, R86, K188, E221, G59, F60, T62, S63, A65, Y67, S93, T124, T126, Y148, F190, D194, 1196, Q200 and/or D214 position/s of SEQ ID NO: 5.

Although enzymes often consist of a large number of amino acids, it is not uncommon that only a comparatively small number of these amino acids is directly involved in catalyzing the chemical reaction. For instance, catalytic triads, which consist of three amino acids, are often found in hydrolase enzymes. An example of a catalytic triad would be the amino acid triad serine-histidine-aspartate in the protease enzyme chymotrypsin. In other enzymes, catalytic tetrades, which consist of four amino acids, are responsible for catalyzing the enzymatic reaction. The chemical identity and structure of these catalytically active amino acids are therefore of particular importance and considerably affect crucial aspects of the reaction such as e.g. the reaction speed or even the product to be formed. It is therefore a preferred embodiment of the invention to provide a method as described herein, wherein the polypeptide comprises a catalytic tetrade consisting of aspartic acid, tyrosine, lysine and histidine or equivalent amino acids. Equivalent amino acids refer to amino acids that are chemically similar and are thus capable of catalyzing the same chemical reactions. A person skilled in the art knows which amino acids are chemically similar. Any amino acid capable of substituting the role of another amino acid in an enzyme reaction is herein considered an equivalent amino acid, as disclosed herein e.g. in Table I. For example, glutamic acid is chemically similar to aspartic acid since both amino acids have an acidic side chain. Therefore, glutamic acid can be an equivalent amino acid to aspartic acid. Tyrosine comprises a hydroxy group in its side chain, which hydroxy group may participate in catalytic reactions e.g. by forming hydrogen bonds. Therefore, amino acids equivalent to tyrosine can be serine and threonine. Lysine and histidine are basic amino acids. Lysine, histidine and arginine can be equivalent amino acids to each other. Also, histidine is known to often participate in enzyme catalysis as proton donor and/or proton acceptor. Therefore, any amino acid capable of substituting for histidine in its role as proton donor and/or proton acceptor can be an equivalent amino acid to histidine. By choosing an enzyme comprising such a catalytic tetrade, the biotransformation of DON at its C8 atom was found to proceed at especially high rates.

In a further preferred embodiment of the invention, means and methods are provided as disclosed herein, wherein the catalytic tetrade consists of D54, Y59, K84 and H125 based on the sequence of SEQ ID NO: 1. Hereby, the inventors found the biotransformation of DON at its C8 atom to work best. D54 refers to an aspartic acid amino acid residue with the position number 54 in the amino acid sequence of SEQ ID NO: 1. Y59 refers to a tyrosine amino acid residue with the position number 59 in the amino acid sequence of SEQ ID NO: 1. K84 refers to a lysine amino acid residue with the position number 84 in the amino acid sequence of SEQ ID NO: 1. H125 refers to a histidine amino acid residue with the position number 125 in the amino acid sequence of SEQ ID NO: 1. It is to be considered that polypeptides other than the polypeptide of SEQ ID NO: 1 can have more or fewer amino acids, leading to a change in the position number of D54, Y59, K84 and H125 of the catalytic tetrade. For example, a polypeptide lacking four N-terminal amino acids compared to the polypeptide of SEQ ID NO: 1 may comprise a catalytic tetrade consisting of D50, Y55, K80 and H121. Also, amino acid deletions or insertions in between two or more amino acids of the catalytic tetrade may result in changes of the position numbers of the amino acids of the catalytic tetrade. Also, two or more amino acids of the amino acids of the catalytic tetrade may switch position. As soon as a polypeptide comprising a catalytic tetrade consisting of aspartic acid, tyrosine, lysine and histidine, is applied in a method as described herein, it is to be regarded to be encompassed by the present invention.

The inventors discovered that biotransformation of DON to a molecule less toxic than DON by transforming DON at the C8 atom can be achieved by applying a method as described above, wherein the biotransformation is performed by a polypeptide having at least 90% sequence identity to the sequence of SEQ ID NO: 1.

In order to reduce or preferably eliminate the adverse health effects observed upon ingestion of feed or food contaminated with DON, the concentration of DON in the feed or food needs to be reduced. Physical or chemical treatment of the feed or food is not feasible for reducing the DON concentration in these matrices. Alternatively, DON can be transformed to another molecule, which has lower toxicity than DON. By providing a method as described herein, wherein the molecule less toxic than deoxynivalenol is at least 5 times less toxic than deoxynivalenol, it is possible to reduce the adverse health effects upon ingestion of the affected food or feed.

In addition to the requirement for a DON biotransformation product molecule to be less toxic than DON, it is crucial for the product molecule to remain stable, i.e. not to be transformed back to the initial molecule DON. Consequently, there is a need for a method that allows biotransformation of DON to a molecule, which is less toxic than DON on the

one hand, and which withstands transformation back to DON in a reverse reaction. This objective was achieved by providing a method as described herein, wherein molecules less toxic than deoxynivalenol are for instance 3,7,8,15-tetrahydroxyscirpene (also referred to as epi-THS), 3,7,8R,15-tetrahydroxyscirpine (THS) or 8-amino-DON. The molecules epi-THS, THS and 8-amino-DON were found by the inventors to be less toxic than DON and surprisingly to withstand a reverse biotransformation reaction back to DON.

For a method of biotransformation of DON to a less toxic molecule in the sense of the present invention to be suitable for industrial application, it is imperative that the catalysis reaction happens sufficiently fast. In a preferred embodiment of the present invention, the polypeptide of the method described herein (e.g., an Akr polypeptide), which polypeptide is contacted with DON, has a turnover number ($k_{cat}$) of at least 1 per second, preferably at least 2 per second, more preferably at least 3 per second, most preferably at least 5 per second in a standard enzymatic activity assay (e.g., an Akr polypeptide). The term "turnover number (kcat)" refers to the maximum number of biotransformation reactions of substrate to product molecules per second by a single catalytic site under given conditions. The unit of kcat is "per second", also written as "$s^{-1}$" or "/s". The term "standard enzymatic activity assay" refers to an assay reaction containing 200 nM Akr enzyme, 1 mM NADPH and DON at a saturating concentration (e.g. at least 2 mM) in Teorell Stenhagen (TS) buffer, pH 6.0 (Ostling and Virtama, Acta Physiologica Scandinavica, 1946, 11:4, 289-293) in a final volume of 200 μL. The assay reaction is performed at 30 °C. The reaction can be started by addition of the Akr enzyme. Samples are taken before reaction start, and 2, 5, 10, 15 and 20 min after reaction start. DON transformation reactions in these samples are stopped by mixing with absolute methanol at a 1:2 ratio. An additional 1:100 dilution is done with 40% methanol. The concentrations of DON and epi-THS in the samples can be determined with liquid chromatography-mass spectrometry (LC-MS) on a Sciex 5500 triple quadrupole mass spectrometer coupled to Agilent 1290 series UHPLC system, using the following MS parameters: Acquisition duration: 4 min; Scan Type: MRM (MRM); Polarity: Negative; Ion Source: Turbo Spray. Analyte parameters for DON are 355.086, 59.0, 20 for the Q1 mass, Q2 mass. Analyte parameters for DON QL (qualifier ion) are 355.086, 265.0, 20 for the Q1 mass, Q2 mass. Analyte parameters for epi-THS are 357.058, 297.2, 20 for the Q1 mass, Q2 mass. And analyte parameters for epi-THS QL (qualifier ion) are 357.058, 177.1, 20 for the Q1 mass, Q2 mass. For LC, eluent A was 5% methanol, 94.9% water (ultrapure), 0.1% acetic acid, and eluent B was 99.9% methanol, 0.1% acetic acid, using a Phenomenex Kinetex 2.6 μm biphenyl 150 x 2.1 mm column, an injection volume of 1 μL and the following mixing protocol at a flow rate of 500 μL/min: At time 0.00 min, 95% eluent A and 5% eluent B; at time 0.10 min, 95% eluent A and 5% eluent B; at time 3.00, 30% eluent A and 70% eluent B%; at time 3.01, 0% eluent A and 100% eluent B; at time 3.30 min, 0% eluent A and 100% eluent B; at time 3.31 min, 95% eluent A and 5% eluent B. Based on the determined concentrations of DON and/or epi-THS at the sampling points throughout the reaction, kinetic parameters such as the turnover number can be calculated by applying standard biochemical methods and calculations.

It is another objective to provide a method for biotransformation of DON as referred to herein, which method can be applied in a broad range of environments. For example, the method should be suitable for application in environments of different pH or at different temperatures. In a further embodiment of the invention, a method is therefore provided as described above, wherein the polypeptide has pH stability within a pH range from at least pH 4.0 to pH 7.0, preferably from at least pH 5.0 to pH 7.0. Hereby, the method can be applied in a broad range of environments. Herein, a polypeptide is considered to have pH stability within a pH range from at least pH 5.0 to pH 7.0 when the polypeptide retains at least 50% of its initial activity after incubation in TS buffer solutions between pH 5.0 and pH 7.0 for 4 h.

Further, by providing a method as described herein, wherein the polypeptide has a temperature stability within a temperature range from at least 30 °C to 40 °C, biotransformation of DON in the sense of the present invention can be performed at a most relevant temperature range. Herein, a polypeptide is considered to have temperature stability within a temperature range from at least 30 °C to 40 °C when the polypeptide retains at least 10% of its initial activity after incubation in TS buffer, pH 6.0, at temperatures between 30 °C and 40 °C for 35 min.

In an embodiment of the present invention, the polypeptide which is contacted with deoxynivalenol, can be a cofactor-dependent oxidoreductase. For a cofactor-dependent oxidoreductase to be able to perform a biotransformation reaction of a target molecule, the cofactor-dependent oxidoreductase needs to also transform a cofactor molecule. Hereby, the cofactor molecule will be consumed and cannot be used in subsequent biotransformation reactions. In such an embodiment of the present invention, the presence of cofactor in amounts sufficient to allow biotransformation of the present DON by the cofactor-dependent oxidoreductase is considered crucial. By providing a method as described herein, wherein an additional polypeptide suitable for cofactor recycling is contacted with the polypeptide performing the biotransformation of DON at the C8 atom of DON, it was found possible to ensure that the cofactor stays available in amounts sufficient to allow biotransformation of the present DON by the cofactor-dependent oxidoreductase. Hereby, the inventors discovered that cofactor depletion by the cofactor-dependent oxidoreductase during the course of the DON biotransformation reaction can be avoided. Herein, a polypeptide is considered suitable for cofactor recycling, when the polypeptide is capable of transforming a consumed cofactor molecule, which was consumed by the cofactor-dependent oxidoreductase during the course of the DON biotransformation at the C8 atom of DON, back to the initial molecule structure of the cofactor, wherein the initial molecule structure of the cofactor can be consumed by the cofactor-dependent

oxidoreductase in a subsequent DON biotransformation reaction. For example, a cofactor-dependent oxidoreductase can depend on the consumption of NADPH as cofactor to be able to perform a biotransformation reaction of DON. The consumption of NADPH by such a cofactor-dependent oxidoreductase leads to the formation of the consumed cofactor molecule, $NADP^+$. An additional polypeptide can be the enzyme glucose-6-phosphate dehydrogenase (G6P-DH). G6P-DH transforms $NADP^+$ back to NADPH, which NADPH can be used in another biotransformation reaction of DON by the cofactor-dependent oxidoreductase. Contacting of the additional polypeptide suitable for cofactor recycling with the polypeptide performing the DON biotransformation may be achieved e.g. by addition of a second polypeptide to the polypeptide performing the DON biotransformation, or by recombinant production of a fusion protein of the two polypeptides, wherein the polypeptide suitable for cofactor recycling and the polypeptide performing the DON biotransformation are covalently linked to one another and form a single molecule. A person having skill in the art knows methods for the production of fusion proteins.

Due to the numerous adverse health effects caused by ingestion of DON, the presence of DON in food or feed is of major concern. The inventors found, that by the use of a polypeptide as described herein for performing a biotransformation of deoxynivalenol at the C8 atom of deoxynivalenol to a molecule less toxic than deoxynivalenol in food or feed, it is possible to obtain food or feed which can be ingested without causing the adverse health effects that would have arisen when the polypeptide would not have been used as described herein.

By providing a food or feed additive comprising a polypeptide having at least 90% sequence identity to the sequence of SEQ ID NO: 1, wherein the polypeptide is capable of performing a biotransformation of deoxynivalenol to a molecule less toxic than deoxynivalenol, and wherein the biotransformation is performed by the polypeptide at the C8 atom of deoxynivalenol, it is possible to prepare food or feed with means that facilitate a reduction or even elimination of DON toxicity. Hereby, DON toxicity may be reduced before, during and/or after ingestion of the feed or food. Also, further components typically comprised in food or feed additives can maximize the beneficial effects a food or feed additive as described herein. Further components can be e.g. additional enzymes, vitamins, amino acids, antioxidants, minerals, antimicrobial compounds, pre- and/or probiotics.

In some embodiments the present invention relates to a polypeptide capable of modifying the C8-atom of a Type B trichothecene, preferably DON or a DON derivative/s, wherein said polypeptide is selected from the group consisting of: (a) a polypeptide having at least 94.1% identity (e.g., at least 94.2%, at least 94.3%, at least 94.4% at least 94.5%, at least 94.6%, at least 94.7%, at least 94.8%, at least 94.9%, at least 95%, at least 96%, at least 97%, at least 98%, at least 99% or 100%) to the polypeptide set forth in SEQ ID NO: 2 or SEQ ID NO: 3; (b) a polypeptide having at least 91.1% identity (e.g., at least 91.2%, at least 91.3%, at least 91.4% at least 91.5%, at least 91.6%, at least 91.7%, at least 91.8%, at least 91.9%, at least 92%, at least 93%, at least 94%, at least 95%, at least 96%, at least 97%, at least 98%, at least 99% or 100%) to the polypeptide set forth in SEQ ID NO: 4; (c) a polypeptide having at least 70% sequence identity (e.g., at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100%) to the mature polypeptide of: SEQ ID NO: 1, SEQ ID NO: 2, SEQ ID NO: 3, SEQ ID NO: 4 or SEQ ID NO: 5; (d) a variant of the polypeptide of SEQ ID NO: 1, SEQ ID NO: 2, SEQ ID NO: 3, SEQ ID NO: 4 or SEQ ID NO: 5, wherein said variant comprising: a substitution, deletion, and/or insertion at one or more positions, wherein said variant having at least 70% (e.g., at least 71 %, at least 72%, at least 73%, at least 74%, at least 75%, at least 76%, at least 77%, at least 78%, at least 79%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 95.5%, at least 96%, at least 96.5%, at least 97%, at least 97.5%, at least 98%, at least 98.5%, at least 99%), but less than 100% sequence identity with the amino acid sequence set forth in SEQ ID NO: 1, SEQ ID NO: 2, SEQ ID NO: 3, SEQ ID NO: 4 or SEQ ID NO: 5; (e) a fragment of the polypeptide of (a)-(d) capable of modifying the C8-atom of a Type B trichothecene, preferably DON or a DON derivative/s.

In some embodiments, Type B trichothecenes of the present invention, preferably DON and DON-derivative/s, are trichothecene compound/s having a carbonyl group (C=O) at the position 8 of the carbon chain (or, in other words, an oxygen atom (=O), doubly bonded to the C8-atom of the carbon chain) and a hydroxyl group (-OH) at the C7-atom of the carbon chain.

In some embodiments a hydroxyl group (-OH) at the C7-atom of a Type B trichothecene of the present invention, preferably DON or a DON derivative/s, remains unchanged during the modification (or transformation) of the present invention. Advantageously, by leaving the C7 atom unchanged the product molecule/compound/intermediate is more stable (e.g., compared to a product molecule with modified C7).

In some embodiments: (i) said modifying is a transformation, e.g. substitution or the change of a functional group of a chemical species (e.g., an oxo-moiety (=O) of the C8-atom of the Type B trichothecene of the present invention, preferably DON or DON derivative, into a different functional group (e.g., a hydroxyl (-OH) or amino (-NH2) moiety); and/or (ii) said modifying is the modifying of only the C8-atom of the Type B trichothecene of the present invention,

preferably DON or DON derivative/s; and/or (iii) said modifying consists of modifying the C8-atom of the Type B trichothecene of the present invention, preferably DON or DON derivative/s; and/or (iv) said modifying is not comprising molecular rearrangement, e.g., not comprising a change in which there is a bond migration of an atom or bond and/or an entering group takes up a different position from the leaving group, with accompanying bond migration; and/or (v) said modifying is not comprising modifying of the C7-atom of the Type B trichothecene of the present invention, preferably DON or DON derivative/s; and/or (vi) said modifying is not comprising isomerization (e.g., intramolecular isomerization) of the Type B trichothecene of the present invention, preferably DON or DON derivative/s, e.g., wherein the product of said modifying is isomeric with a corresponding reactant, e.g., the Type B trichothecene of the present invention, preferably DON or DON derivative.

In some embodiments a Type B trichothecene of the present invention, preferably DON derivative of the present invention, is selected from the group consisting of: 3-Acetyldeoxynivalenol, 15-Acetyldeoxynivalenol, Nivalenol, 4-acetylnivalenol, 3-amino Deoxynivalenol.

In some embodiments of the present invention: (i) said modifying of the C8-atom is one or more of the following: reducing a keto moiety (preferably reducing said keto moiety to a hydroxyl moiety), transaminating a keto moiety, detoxifying and/or reducing the toxicity of a Type B trichothecene of the present invention, preferably DON or a DON derivative/s.

In some embodiments of the present invention said modifying the C8-atom is a reduction and/or transamination of the C8-atom leading to one or more of the following: 3,7,8,15-tetrahydroxyscirpene (epi-THS), 3,7,8R,15-tetrahydroxyscirpine (THS), 8-amino-DON, 8-amino-3-ketoDON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-keto Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol.

In some embodiments the polypeptide of the present invention comprises: an amino acid D or an equivalent amino acid at a position corresponding to position 54 of SEQ ID NO: 1; Y or an equivalent amino acid at a position corresponding to position 59 of SEQ ID NO: 1; K or an equivalent amino acid at a position corresponding to position 84 of SEQ ID NO: 1 and H or an equivalent amino acid at a position corresponding to position 125 of SEQ ID NO: 1, preferably using the numbering of SEQ ID NO: 1.

In some embodiments the polypeptide of the present invention comprises an $(\alpha/\beta)_8$-barrel structure (e.g., SEQ ID NOs:1-3).

In some embodiments the polypeptide of the present invention has dehydrogenase and/or transaminase activity.

In some embodiments of the present invention said dehydrogenase activity is the dehydrogenase activity having an EC: 1.1.1.- activity; and/or said transaminase activity is the transaminase activity having an EC: 2.6.1.- activity.

In some embodiments of the present invention said dehydrogenase activity comprises or consists of alcohol dehydrogenase (NADP(+)) activity having an EC: 1.1.1.2 activity.

In some embodiments the polypeptide of the present invention is capable of altering the level of the Type B trichothecene of the present invention, preferably DON and/or a DON derivative/s, preferably reducing said level, and/or detoxifying the Type B trichothecene of the present invention, preferably DON and/or DON derivatives.

In some embodiments the polypeptide of the present invention has a turnover number (kcat) of at least 1 per second, preferably at least 2 per second, more preferably at least 3 per second, most preferably at least 5 per second, e.g., in an enzymatic activity assay.

In some embodiments the polypeptide of the present invention is stable within pH range from 5.0 to about 7.0, preferably from 4.0 to about 7.0.

In some embodiments the polypeptide of the present invention is stable within a temperature range from about 30 °C to about 40 °C.

In some embodiments the present invention relates to a polynucleotide encoding the polypeptide of the present invention.

In some embodiments the present invention relates to a nucleic acid construct or expression vector capable of expressing the polynucleotide of the present invention, wherein said nucleic acid construct or said expression vector comprising the polynucleotide of the present invention, preferably said polynucleotide is operably linked to one or more control sequences that direct the production of the polypeptide in an expression host.

In some embodiments the present invention relates to a recombinant host cell (e.g., an isolated recombinant host cell) comprising one or more of the following: (i) one or more polypeptides of the present invention; (ii) one or more polynucleotide/s of the present invention; (iii) one or more nucleic acid construct/s and/or expression vector/s of the present invention.

In some embodiments the recombinant host cell of the present invention is selected from a bacterial cell, yeast cell, mammalian cell, insect cell and plant cell, preferably from a bacterial cell and yeast cell.

In some embodiments the recombinant host cell of the present invention is comprised within a recombinant

plant seed.

In some embodiments the recombinant plant host cell of the present invention is homozygous for said (ii) and/or (iii).

In some embodiments the present invention relates to a transgenic plant, transgenic seed or transgenic pollen grain comprising one or more (e.g., plurality) of the following: (i) one or more polypeptides of the present invention; (ii) one or more polynucleotide of the present invention; (iii) one or more nucleic acid constructs and/or expression vectors of the present invention; and/or (iv) one or more recombinant host cell of the present invention.

In some embodiments the transgenic plant, transgenic seed or transgenic pollen grain of the present invention is capable of producing one or more polypeptides of the present invention, preferably said transgenic plant, transgenic seed or transgenic pollen grain is genetically-modified to produce one or more polypeptide of the present invention.

In some embodiments of the present invention said transgenic plant, transgenic seed or transgenic pollen grain is homozygous for said (ii) and/or (iii).

In some embodiments the present invention relates to foodstuff, intermediate foodstuff; fodder, an intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic and/or mixture/s thereof, comprising one or more of the following: polypeptide/s, polynucleotide/s, nucleic acid construct/s, expression vector/s, recombinant host cell/s, transgenic plant, transgenic seed and/or transgenic pollen grain of the present invention.

In some embodiments the present invention relates to a composition or kit comprising one or more of the following: polypeptide/s, polynucleotide/s, nucleic acid construct/s, expression vector/s, recombinant host cell/s, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement, prebiotic, intermediate prebiotic and/or mixture/s thereof of the present invention.

In some embodiments of the present invention said composition or kit is one or more of the following: a pharmaceutical-, veterinary-, diagnostic-, detoxifying-, seed treatment-, monitoring- and/or screening composition or kit.

In some embodiments of the present invention said composition or kit comprising: a cofactor recycling system, preferably the cofactor recycling system comprises a second polypeptide, wherein said second polypeptide is suitable for converting NADP+ to NADPH or NAD+ to NADH, further preferably the cofactor recycling system further comprises a molecule selected from the group consisting of glucose-6-phosphate.

In some embodiments of the present invention relates to a compound or intermediate (a Type B trichothecene of the present invention modified at C8-atom) selected from the group consisting of: epi-THS, THS, 8-amino-DON, 8-amino-3-keto-DON, 8-amino-3-ketoDON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-keto Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol.

In some embodiments of the present invention relates to a method for producing the compound or intermediate (a Type B trichothecene of the present invention modified at C8-atom) selected from the group consisting of: epi-THS, THS, 8-amino-DON, 8-amino-3-keto-DON, 8-Hydroxy-3-keto-Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino-Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol, said method comprising: applying one or more polypeptide/s, recombinant cell/s, composition/s and/or kit/s of the present inventions to the Type B trichothecene of the present invention, preferably DON and/or a DON derivative/s.

In some embodiments a compound or intermediate of the present invention (a Type B trichothecene of the present invention modified at C8-atom) is selected from the group consisting of: epi-THS, THS, 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol, produced by the method of producing the compound or intermediate of the present invention.

In some embodiments the present invention relates to a method for one or more of the following, said method is carried out (or suitable to be carried out) in a substrate or sample containing the Type B trichothecene of the present invention, preferably DON and/or a DON derivative/s: (i) method for modifying the C8-atom of the Type B trichothecene of the present invention, preferably DON and/or a DON derivative/s; (ii) method for altering the level of the Type B trichothecene of the present invention, preferably DON and/or a DON derivative/s (e.g., reducing the level) and/or detoxifying the Type B trichothecene of the present invention, preferably DON and/or DON derivative/s; (iii) method for

bio-transforming (e.g., enzymatically modifying) the Type B trichothecene of the present invention, preferably DON and/or DON derivative/s, preferably said bio-transforming is enzymatic; (iv) method for detecting and/or assessing the level of mycotoxin; (v) method for reducing the toxicity of the Type B trichothecene of the present invention, preferably DON and/or a DON derivative/s and/or for treating seeds; said method comprising: (a) providing one or more of the following: polypeptide/s, compound/s, intermediate/s, polynucleotide/s, nucleic acid construct/s, expression vector/s, recombinant host cell/s, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit of the present invention; (b) applying (a) to said substrate or sample; (c) preferably, detecting and/or quantifying one or more compound/s or intermediate/s of the present invention (e.g., the Type B trichothecene of the present invention modified at C8-atom).

In some embodiments said modifying the C8-atom is reducing, transaminating, detoxifying and/or reducing the toxicity of the Type B trichothecene of the present invention, preferably DON and/or a DON derivative/s.

In some embodiments said modifying C8-atom is producing one or more of the following: epi-THS, THS, 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol.

In some embodiments said method is a method of manufacture of biogas, bioethanol, silage or sugar, preferably from sugar cane or sugar beets.

In some embodiments said substrate is selected from the group consisting of: agrarian product, crop, Distiller's Dried Grains with Solubles (DDGS), corn, sugar cane or sugar beets.

In some embodiments the present invention relates to a method for treatment, amelioration, prophylaxis and/or diagnostics of mycotoxicosis (e.g., in a subject or animal), comprising: (i) providing: a therapeutically efficient amount of one or more of the following: polypeptide/s, polynucleotide/s, nucleic acid construct/s, expression vector/s, recombinant host cell/s, transgenic plant, transgenic seed, transgenic pollen grain, composition/s or kit/s of the present invention; (ii) administering said therapeutically efficient amount as defined in (i) (e.g., to said subject or animal).

In some embodiments said method produces one or more of the molecule/s, compound/s or intermediate/s (e.g., the Type B trichothecene of the present invention modified at C8-atom) selected from the group consisting of: epi-THS, THS, amino-DON and/or 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol. In some further embodiments said one or more molecule/s, compound/s and/or intermediate/s can be used e.g., as a reference standard e.g. to evaluate the success/progress of a method for detoxifying a corresponding mycotoxin according to the present invention

In some embodiments said method further comprising: providing a cofactor recycling system, preferably the cofactor recycling system comprises a second polypeptide, wherein the second polypeptide is suitable for converting NADP+ to NADPH or NAD+ to NADH, further preferably the cofactor recycling system further comprises a molecule selected from the group consisting of glucose-6-phosphate.

In some embodiments the polypeptide, compound, intermediate, polynucleotide, nucleic acid construct, expression vector, recombinant host cell, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit of the present invention, is suitable for use in therapy, prophylaxis and/or as a medicament (e.g., for veterinary use).

In some embodiments the polypeptide, compound or intermediate, polynucleotide, nucleic acid construct, expression vector, recombinant host cell, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit of the present invention, is suitable for use in one or more of the following methods: (i) method for modifying C8-atom of the Type B trichothecene of the present invention, preferably DON and/or a DON derivative/s; (ii) method for detoxifying the Type B trichothecene of the present invention, preferably DON and/or a DON derivative/s, having a keto moiety (C=O) at the C-8 atom; (iii) method for bio-transforming the Type B trichothecene of the present invention, preferably DON and/or a DON derivative/s, having a keto moiety (C=O) at the C-8 atom, preferably said bio-transforming

is enzymatic; (iv) method for treatment, amelioration, prophylaxis and/or diagnostics of the Type B trichothecene mycotoxicosis, preferably DON mycotoxicosis; (v) method for monitoring development of mycotoxicosis and/or assessing the efficacy of the Type B trichothecene mycotoxicosis, prophylaxis and/or therapy, preferably DON-mycotoxicosis prophylaxis and/or therapy; (vi) method for screening a candidate compound for the Type B trichothecene detoxification activity, preferably DON detoxification activity; (vii) method for detecting and/or assessing the level of the Type B trichothecene, preferably DON and/or a DON derivative/s; (viii) method for reducing the toxicity of the Type B trichothecene, preferably DON and/or a DON derivative/s; (ix) method for altering, preferably reducing, the level of the Type B trichothecene, preferably DON and/or a DON derivative/s; (x) producing one or more of the following: foodstuff, intermediate foodstuff; fodder, intermediate fodder; feed intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic; pharmaceutical, veterinary, diagnostic, detoxifying, monitoring and/or screening composition or kit; (xi) any method of the present invention; (xii) any combination of methods according to (i)-(xi); (xiii) method of any of (i)-(xii), wherein said method is an *in vitro, ex vivo* or *in vivo* method.

In some embodiments the present invention relates to a use of one or more polypeptide/s, compound/s, intermediate/s, polynucleotide/s, nucleic acid construct/s, expression vector/s, recombinant host cell/s, transgenic plant/s, transgenic seed/s, transgenic pollen grain/s, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit of the present invention for/in one or more of the following: (i) for modifying the C8-atom of DON and/or DON derivative/s; (ii) for detoxifying the Type B trichothecene, preferably DON and/or DON derivative/s; (iii) for bio-transforming of the Type B trichothecene, preferably DON and/or DON derivative/s having a keto moiety (C=O) at the C8-atom, preferably said bio-transforming is enzymatic; (iv) for treatment, amelioration, prophylaxis and/or diagnostics of the Type B trichothecene mycotoxicosis, preferably DON-mycotoxicosis; (v) for monitoring development of mycotoxicosis and/or assessing the efficacy of the Type B trichothecene mycotoxicosis prophylaxis and/or therapy; preferably DON-mycotoxicosis prophylaxis and/or therapy; (vi) for screening a candidate compound for anti-mycotoxicosis activity; (vii) for detecting and/or assessing the level of the Type B trichothecene, preferably DON and/or DON derivative/s; (viii) for inhibiting toxicity of the Type B trichothecene, preferably DON and/or DON derivative/s; (ix) for altering, preferably reducing the level of the Type B trichothecene, preferably DON and/or DON derivative/s; (x) for producing one or more of the following: a foodstuff, intermediate foodstuff; fodder, an intermediate fodder; feed, intermediate feed; additive (e.g., foodstuff-, fodder- or feed additive), intermediate additive (e.g., foodstuff-, fodder- or feed intermediate additive); detoxifying agent, an intermediate detoxifying agent; nutritional supplement, an intermediate nutritional supplement; prebiotic, intermediate prebiotic; and/or pharmaceutical-diagnostic-, detoxifying-, monitoring- and/or screening composition or kit; (xi) in any method of the present invention; (xii) any combination of (i)-(xi); (xiii) use according to any one (i)-(xii), wherein said use is an *in vitro, ex vivo* or *in vivo* use.

In some embodiments reducing the toxicity of the Type B trichothecene mycotoxin (e.g., DON or DON derivative) means including transformation (or modification) of said mycotoxin to a reaction product less toxic than said mycotoxin (e.g., DON or DON derivative).

In some embodiments the present invention relates to an Akr I polypeptide (variant) of the present invention comprising one or more equivalent (or same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283 and/or L284, preferably to D54, Y59, K84 and/or H125 position/s of SEQ ID NO: 1.

In some embodiments the present invention relates to an Akr II polypeptide (variant) of the present invention comprising one or more equivalent (or same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283 and/or L284, preferably to D54, Y59, K84 and/or H125 position/s of SEQ ID NO: 2.

In some embodiments the present invention relates to an Akr III polypeptide (variant) of the present invention comprising one or more equivalent (or same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283 and/or L284 preferably to D54, Y59, K84 and/or H125 position/s of SEQ ID NO: 3.

In some embodiments the present invention relates to an ADH-Lk polypeptide (variant) of the present invention comprising one or more equivalent (or same) amino acid/s in position/s corresponding to: N114, S143, Y156 and/or K160 position/s of SEQ ID NO: 4; and/or G14, H17, G18, 119, G20, G38, R39, R40, H62, D63, V64, N90, A91, G92, I93, V113, M141, S142, S143, Y156, K160, P188, G189, P190, 1191, T193, P194 and/or L195 position/s of SEQ ID NO: 4; and/or E67, V99, E100, T102, T104, W107, R108, L111, L115, D116, F119, F120, T122, R123, 1126, Q127, K130, G146, Q147, G149, D150, P151, G154, S157, A158, G161, A162, R164, 1165, M166, K168, S169, A170, L172, L173 and/or C174 position/s of SEQ ID NO: 4; and/or R4, E67, V99, E100, T102, T104, W107, R108, L111, L115, D116, F119, F120, T122, R123, 1126, Q127, K130, G146, Q147, V148, G149, D150, P151, G154, S157, A158, G161, A162,

R164, 1165, M166, K168, S169, A170, A171, L172, L173, C174, A175, P190, T212, P213, M214, G215, H216, 1217, G218, E219, D222, W225, V226, Y229, E234, K236, F237, A238, T239, G240, A241, E242, F243, V244, V245, D246, G247, G248, Y249, T250, A251 and/or Q252 position/s of SEQ ID NO: 4.

In some embodiments the present invention relates to a TAM-Ac polypeptide (variant) of the present invention comprising one or more equivalent (or same) amino acid/s in position/s corresponding to: K188, Y67, R86, K188, E221, I246, T247, T283, R86, K188, E221, G59, F60, T62, S63, A65, Y67, S93, T124, T126, Y148, F190, D194, 1196, Q200 and/or D214 position/s of SEQ ID NO: 5.

It should be understood that this invention is not limited to the particular methodology, protocols, and reagents, etc., described herein and as such can vary. The terminology used herein is for the purpose of describing particular embodiments only, and is not intended to limit the scope of the present invention, which is defined solely by the claims.

All publications and patents cited throughout the text of this specification (including all patents, patent applications, scientific publications, manufacturer's specifications, instructions, etc.), whether supra or infra, are hereby incorporated by reference in their entirety. Nothing herein is to be construed as an admission that the invention is not entitled to antedate such disclosure by virtue of prior invention. To the extent the material incorporated by reference contradicts or is inconsistent with this specification, the specification will supersede any such material.

**The invention** is **also characterized by the following items:**

1. A polypeptide capable of modifying the C8-atom of a Type B trichothecene, preferably DON or a DON derivative/s, further preferably wherein a hydroxyl group (-OH) at the C7-atom of the Type B trichothecene (e.g., DON or a DON derivative/s) remains unchanged during said modification; wherein said polypeptide is one or more of the following:

    a) a polypeptide having at least 94.1% identity (e.g., at least 94.2%, at least 94.3%, at least 94.4% at least 94.5%, at least 94.6%, at least 94.7%, at least 94.8%, at least 94.9%, at least 95%, at least 96%, at least 97%, at least 98%, at least 99% or 100%) to the polypeptide set forth in SEQ ID NO: 2 or SEQ ID NO: 3;
    b) a polypeptide having at least 91.1% identity (e.g., at least 91.2%, at least 91.3%, at least 91.4% at least 91.5%, at least 91.6%, at least 91.7%, at least 91.8%, at least 91.9%, at least 92%, at least 93%, at least 94%, at least 95%, at least 96%, at least 97%, at least 98%, at least 99% or 100%) to the polypeptide set forth in SEQ ID NO: 4;
    c) a polypeptide having at least 70% sequence identity (e.g., at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100%) to the mature polypeptide of: SEQ ID NO: 1, SEQ ID NO: 2, SEQ ID NO: 3, SEQ ID NO: 4 or SEQ ID NO: 5;
    d) a variant of the polypeptide of SEQ ID NO: 1, SEQ ID NO: 2, SEQ ID NO: 3, SEQ ID NO: 4 or SEQ ID NO: 5, wherein said variant comprising a substitution, deletion, and/or insertion at one or more positions, wherein said variant having at least 70% (e.g., at least 71 %, at least 72%, at least 73%, at least 74%, at least 75%, at least 76%, at least 77%, at least 78%, at least 79%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 95.5%, at least 96%, at least 96.5%, at least 97%, at least 97.5%, at least 98%, at least 98.5%, at least 99%), but less than 100% sequence identity with the amino acid sequence set forth in SEQ ID NO: 1, SEQ ID NO: 2, SEQ ID NO: 3, SEQ ID NO: 4 or SEQ ID NO: 5;
    e) a fragment of the polypeptide of (a)-(d) capable of modifying the C8-atom of the Type B trichothecene, preferably DON or a DON derivative/s.

2. The polypeptide according to any one of the preceding items, wherein said polypeptide comprises one or more equivalent (e.g., same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283, L284, D54, Y59, K84 and/or H125 position/s of SEQ ID NO: 1 (preferably using the numbering of SEQ ID NO: 1).
3. The polypeptide according to any one of the preceding items, wherein said polypeptide comprises one or more equivalent (e.g., same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283, L284, D54, Y59, K84 and/or H125 position/s of SEQ ID NO: 2 (preferably using the numbering of SEQ ID NO: 1).
4. The polypeptide according to any one of the preceding items, wherein said polypeptide comprises one or more equivalent (e.g., same) amino acid/s in position/s corresponding to: G18, A19, G20, D54, Y59, K84, H125, D126, A160, G161, Y182, A209, G210, P211, Y212, A213, S214, G215, R255, V272, V273, G274, L275, R280, A283, L284, D54, Y59, K84 and/or H125 position/s of SEQ ID NO: 3 (preferably using the numbering of SEQ ID NO: 1).
5. The polypeptide according to any one of the preceding items, wherein said polypeptide comprises one or more

equivalent (e.g., same) amino acid/s in position/s corresponding to: N114, S143, Y156 and/or K160 position/s of SEQ ID NO: 4; and/or G14, H17, G18, 119, G20, G38, R39, R40, H62, D63, V64, N90, A91, G92, I93, V113, M141, S142, S143, Y156, K160, P188, G189, P190, 1191, T193, P194 and/or L195 position/s of SEQ ID NO: 4; and/or E67, V99, E100, T102, T104, W107, R108, L111, L115, D116, F119, F120, T122, R123, 1126, Q127, K130, G146, Q147, G149, D150, P151, G154, S157, A158, G161, A162, R164, 1165, M166, K168, S169, A170, L172, L173 and/or C174 position/s of SEQ ID NO: 4; and/or R4, E67, V99, E100, T102, T104, W107, R108, L111, L115, D116, F119, F120, T122, R123, 1126, Q127, K130, G146, Q147, V148, G149, D150, P151, G154, S157, A158, G161, A162, R164, 1165, M166, K168, S169, A170, A171, L172, L173, C174, A175, P190, T212, P213, M214, G215, H216, 1217, G218, E219, D222, W225, V226, Y229, E234, K236, F237, A238, T239, G240, A241, E242, F243, V244, V245, D246, G247, G248, Y249, T250, A251 and/or Q252 position/s of SEQ ID NO: 4 (preferably using the numbering of SEQ ID NO: 4).

6. The polypeptide according to any one of the preceding items, wherein said polypeptide comprises one or more equivalent (e.g., same) amino acid/s in position/s corresponding to: K188, Y67, R86, K188, E221, I246, T247, T283, R86, K188, E221, G59, F60, T62, S63, A65, Y67, S93, T124, T126, Y148, F190, D194, 1196, Q200 and/or D214 position/s of SEQ ID NO: 5 (preferably using the numbering of SEQ ID NO: 5).

7. The polypeptide according to any one of the preceding items, wherein: (i) said modifying of the C8-atom is one or more of the following: reducing a carbonyl moiety (e.g., keto moiety) (preferably reducing said keto moiety to a hydroxyl moiety), transaminating a carbonyl moiety (e.g., a keto moiety), detoxifying and/or reducing the toxicity of the Type B trichothecene, preferably DON or a DON derivative/s; and/or (ii) said Type B trichothecene, preferably the DON derivative is selected from the group consisting of: 3-Acetyldeoxynivalenol, 15-Acetyldeoxynivalenol, Nivalenol, 4-acetylnivalenol, 3-amino Deoxynivalenol.

8. The polypeptide according to any one of the preceding items, wherein said modifying the C8-atom is a reduction and/or transamination of the C8-atom leading to one or more of the following: epi-THS, THS, 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol.

9. The polypeptide according to any one of the preceding items, comprising: an amino acid D or an equivalent amino acid at a position corresponding to position 54 of SEQ ID NO: 1; Y or an equivalent amino acid at a position corresponding to position 59 of SEQ ID NO: 1; K or an equivalent amino acid at a position corresponding to position 84 of SEQ ID NO: 1 and H or an equivalent amino acid at a position corresponding to position 125 of SEQ ID NO: 1, preferably using the numbering of SEQ ID NO: 1.

10. The polypeptide according to any one of the preceding items, comprising an $(\alpha/\beta)_8$-barrel structure (e.g., SEQ ID NOs: 1-3).

11. The polypeptide according to any one of the preceding items, wherein said polypeptide has dehydrogenase and/or transaminase activity.

12. The polypeptide according to any one of the preceding items, wherein said dehydrogenase activity is the dehydrogenase activity having an EC: 1.1.1.- activity; and/or said transaminase activity is the transaminase activity having an EC: 2.6.1.-activity.

13. The polypeptide according to any one of the preceding items, wherein said dehydrogenase activity comprises or consists of alcohol dehydrogenase (NADP(+)) activity having an EC: 1.1.1.2 activity.

14. The polypeptide according to any one of the preceding items, wherein said polypeptide is capable of altering the level of a Type B trichothecene, preferably DON and/or DON derivatives, preferably reducing said level, and/or detoxifying a Type B trichothecene, preferably DON and/or DON derivatives.

15. The polypeptide according to any one of the preceding items, wherein said polypeptide has a turnover number (kcat) of at least 1 per second, preferably at least 2 per second, more preferably at least 3 per second, most preferably at least 5 per second, e.g., in an enzymatic activity assay.

16. The polypeptide according to any one of the preceding items, wherein said polypeptide is stable within pH range from 5.0 to about 7.0, preferably from 4.0 to about 7.0.

17. The polypeptide according to any one of the preceding items, wherein said polypeptide is stable within a temperature range from about 30 °C to about 40 °C.

18. A polynucleotide encoding the polypeptide according to any one of the preceding items.

19. A nucleic acid construct or expression vector capable of expressing the polynucleotide according to any one of the preceding items, wherein said nucleic acid construct or said expression vector comprising the polynucleotide according to any one of the preceding items, preferably said polynucleotide is operably linked to one or more control sequences that direct the production of the polypeptide in an expression host.

20. A recombinant host cell (e.g., an isolated recombinant host cell) comprising one or more of the following:

i) one or more polypeptides according to any one of the preceding items;
ii) one or more polynucleotide according to any one of the preceding items;
iii) one or more nucleic acid constructs and/or expression vectors according to any one of the preceding items.

21. The recombinant host cell according to any one of the preceding items, wherein the recombinant host cell is selected from a bacterial cell, yeast cell, mammalian cell, insect cell and plant cell, preferably from a bacterial cell and yeast cell.
22. The recombinant host cell according to any one of the preceding items, wherein said recombinant host cell is comprised within a recombinant plant seed.
23. The recombinant plant host cell according to any one of the preceding items, wherein said recombinant plant host cell is homozygous for said (ii) and/or (iii).
24. A transgenic plant, transgenic seed or transgenic pollen grain comprising one or more (e.g., plurality) of the following:

i) one or more polypeptides according to any one of the preceding items;
ii) one or more polynucleotide according to any one of the preceding items;
iii) one or more nucleic acid constructs and/or expression vectors according to any one of the preceding items; and/or
iv) one or more recombinant host cell according to any one of the preceding items.

25. The transgenic plant, transgenic seed or transgenic pollen grain according to any one of the preceding items, capable of producing one or more polypeptides according to any one of the preceding items, preferably said transgenic plant, transgenic seed or transgenic pollen grain is genetically-modified to produce one or more polypeptide according to any one of the preceding items.
26. The transgenic plant, transgenic seed or transgenic pollen grain according to any one of the preceding items, wherein said transgenic plant, transgenic seed or transgenic pollen grain is homozygous for said (ii) and/or (iii).
27. A foodstuff, intermediate foodstuff; fodder, an intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic and/or mixture/s thereof, comprising one or more of the following: polypeptide/s, polynucleotide/s, nucleic acid construct/s, expression vector/s and/or recombinant host cell/s, transgenic plant, transgenic seed and/or transgenic pollen grain according to any one of the preceding items.
28. A composition or kit comprising one or more of the following: polypeptide/s, polynucleotide/s, nucleic acid construct/s, expression vector/s and/or recombinant host cell/s, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement, prebiotic, intermediate prebiotic and/or mixture/s thereof according to any one of the preceding items.
29. The composition or kit according to any one of the preceding items, wherein said composition or kit is one or more of the following: a pharmaceutical-, veterinary-diagnostic-, detoxifying-, seed treatment-, monitoring- and/or screening composition or kit.
30. The composition or kit according to any one of the preceding items, comprising: a cofactor recycling system, preferably the cofactor recycling system comprises a second polypeptide, wherein said second polypeptide is suitable for converting NADP+ to NADPH or NAD+ to NADH, further preferably the cofactor recycling system further comprises a molecule selected from the group consisting of glucose-6-phosphate.
31. A compound or intermediate (e.g., a Type B trichothecene modified at the C8-atom) selected from the group consisting of: 3,7,8,15-tetrahydroxyscirpene (epi-THS), 3,7,8R,15-tetrahydroxyscirpine (THS), 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and 8-Amino-3-amino Deoxynivalenol.
32. A method for producing the compound or intermediate (e.g., a Type B trichothecene modified at the C8-atom) selected from the group consisting of: epi-THS, THS, 8-amino-DON and/or 8-amino-3-keto-DON, said method comprising: applying one or more polypeptide/s, recombinant cell/s, composition/s and/or kit/s according to any one of the preceding items to a Type B trichothecene, preferably DON and/or DON derivatives; preferably, wherein hydroxyl group (-OH) at the C7-atom of DON or a DON derivative/s remains unchanged during the modification.
33. A compound or intermediate (e.g., a Type B trichothecene modified at the C8-atom) selected from the group consisting of: epi-THS, THS, 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 88-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and 8-Amino-3-amino Deoxynivalenol, produced by the method of producing the compound or intermediate according to any one of the preceding items.

34. A method for one or more of the following, said method carried out in a substrate or sample comprising a Type B trichothecene, preferably DON and/or a DON derivative/s:

i) for modifying the C8-atom of the Type B trichothecene, preferably DON and/or a DON derivative/s;
ii) for altering the level of the Type B trichothecene, preferably DON and/or a DON derivative/s (e.g., reducing the level) and/or detoxifying the Type B trichothecene, preferably DON and/or DON derivative/s;
iii) for bio-transforming (e.g., enzymatically modifying) the Type B trichothecene, preferably DON and/or DON derivative/s, preferably said bio-transforming is enzymatic;
iv) for detecting and/or assessing the level of mycotoxin; and/or
v) for reducing the toxicity of the Type B trichothecene, preferably DON and/or a DON derivative/s and/or for treating seeds;

said method comprising:

(a) providing one or more of the following: polypeptide/s, compound/s, intermediate/s, polynucleotide/s, nucleic acid construct/s, expression vector/s, recombinant host cell/s, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit according to any one of the preceding items;
(b) applying (a) to said substrate or sample;
(c) preferably, detecting and/or quantifying one or more compound/s or intermediate/s according to any one of the preceding items.

35. The method according to any one of the preceding items, wherein said modifying C8-atom is detoxifying and/or reducing the toxicity of the Type B trichothecene, preferably DON and/or a DON derivative/s; preferably wherein hydroxyl group (-OH) at the C7-atom of the Type B trichothecene, preferably DON or a DON derivative/s, remains unchanged during the modification.

36. The method according to any one of the preceding items, wherein said modifying C8-atom is producing one or more of the following: epi-THS, THS, 8-amino-DON, 8-amino-3-ketoDON, 8-amino-3-ketoDON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-keto Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol.

37. The method according to any one of the preceding items, wherein said method is a method of manufacture of biogas, bioethanol, silage or sugar, preferably from sugar cane or sugar beets.

38. The method according to any one of the preceding items, wherein said substrate is selected from the group consisting of: agrarian product, crop, Distiller's Dried Grains with Solubles (DDGS), corn, sugar cane or sugar beets.

39. A method for treatment, amelioration, prophylaxis and/or diagnostics of mycotoxicosis (e.g., in a subject or animal), comprising:

i) providing: a therapeutically efficient amount of one or more of the following: polypeptide/s, polynucleotide/s, nucleic acid construct/s, expression vector/s, recombinant host cell/s, transgenic plant, transgenic seed, transgenic pollen grain, composition/s or kit/s according to any one of the preceding items;
ii) administering said therapeutically efficient amount as defined in (i) (e.g., to said subject or animal).

40. The method according to any one of the preceding items, wherein said method produces one or more of the molecule/s, compound/s or intermediate/s (e.g., a Type B trichothecene, e.g., modified at the C8-atom) selected from the group consisting of: epi-THS, THS, 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy-Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and

8-Amino-3-amino Deoxynivalenol.

41. The method according to any one of the preceding items, further comprising: providing a cofactor recycling system, preferably the cofactor recycling system comprises a second polypeptide, wherein the second polypeptide is suitable for converting NADP+ to NADPH or NAD+ to NADH, further preferably the cofactor recycling system further comprises a molecule selected from the group consisting of glucose-6-phosphate.

42. The polypeptide, compound, intermediate, polynucleotide, nucleic acid construct, expression vector, recombinant host cell, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit according to any one of the preceding items, for use in therapy, prophylaxis and/or as a medicament (e.g., for veterinary use).

43. The polypeptide, compound or intermediate, polynucleotide, nucleic acid construct, expression vector, recombinant host cell, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit according to any one of the preceding items, for use in one or more of the following methods:

i) method for modifying C8-atom of a Type B trichothecene, preferably DON and/or a DON derivative/s;
ii) method for detoxifying a Type B trichothecene, preferably DON and/or a DON derivative/s having a carbonyl moiety (e.g., keto moiety) (C=O) at the C-8 atom;
iii) method for bio-transforming a Type B trichothecene, preferably DON and/or a DON derivative/s having a carbonyl moiety (e.g., keto moiety) (C=O) at the C-8 atom, preferably said bio-transforming is enzymatic;
iv) method for treatment, amelioration, prophylaxis and/or diagnostics of a Type B trichothecene mycotoxicosis, preferably DON-mycotoxicosis;
v) method for monitoring development of mycotoxicosis and/or assessing the efficacy of Type B trichothecene mycotoxicosis prophylaxis and/or therapy, preferably DON-mycotoxicosis prophylaxis and/or therapy;
vi) method for screening a candidate compound for Type B trichothecene detoxification activity, preferably DON detoxification activity;
vii) method for detecting and/or assessing the level of Type B trichothecene, preferably DON and/or a DON derivative/s;
viii) method for reducing the toxicity of Type B trichothecene, preferably DON and/or a DON derivative/s;
ix) method for altering, preferably reducing, the level of Type B trichothecene, preferably DON and/or a DON derivative/s;
x) producing one or more of the following: foodstuff, intermediate foodstuff; fodder, intermediate fodder; feed intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic; pharmaceutical, veterinary, diagnostic, detoxifying, monitoring and/or screening composition or kit;
xi) method according to any one of the preceding items, preferably wherein hydroxyl group (-OH) at the C7-atom of Type B trichothecene, preferably DON or a DON derivative/s, remains unchanged during the modification;
xii) any combination of methods according to (i)-(xi);
xiii) method of any of (i)-(xii), wherein said method is an *in vitro, ex vivo* or *in vivo* method.

44. Use of one or more polypeptide/s, compound/s, intermediate/s, polynucleotide/s, nucleic acid construct/s, expression vector/s, recombinant host cell/s, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit according to any one of the preceding items for/in one or more of the following:

i) for modifying the C8-atom of a Type B trichothecene, preferably DON and/or DON derivative/s;
ii) for detoxifying a Type B trichothecene, preferably DON and/or DON derivative/s;
iii) for bio-transforming of a Type B trichothecene, preferably DON and/or DON derivative/s having a carbonyl moiety (e.g., keto moiety) (C=O) at the C8-atom, preferably said bio-transforming is enzymatic;
iv) for treatment, amelioration, prophylaxis and/or diagnostics of a Type B trichothecene mycotoxicosis, preferably DON-mycotoxicosis;
v) for monitoring development of mycotoxicosis and/or assessing the efficacy of Type B trichothecene mycotoxicosis prophylaxis and/or therapy, preferably DON-mycotoxicosis prophylaxis and/or therapy;
vi) for screening a candidate compound for anti-mycotoxicosis activity;
vii) for detecting and/or assessing the level of a Type B trichothecene, preferably DON and/or DON derivative/s;
viii) for inhibiting toxicity of a Type B trichothecene, preferably DON and/or DON derivative/s;
ix) for altering, preferably reducing, the level of a Type B trichothecene, preferably DON and/or DON derivative/s;
x) for producing one or more of the following: a foodstuff, intermediate foodstuff; fodder, an intermediate fodder; feed, intermediate feed; additive (e.g., foodstuff-, fodder- or feed additive), intermediate additive (e.g., foodstuff-, fodder- or feed intermediate additive); detoxifying agent, an intermediate detoxifying agent; nutritional supplement, an intermediate nutritional supplement; prebiotic, intermediate prebiotic; and/or pharmaceutical- diagnostic-, detoxifying-, monitoring- and/or screening composition or kit.
xi) in the method according to any one of the preceding items, preferably wherein hydroxyl group (-OH) at the C7-atom of a Type B trichothecene, preferably DON or a DON derivative/s, remains unchanged during the modification;
xii) any combination of (i)-(xi);
xiii) use according to any one (i)-(xii), wherein said use is an *in vitro, ex vivo* or *in vivo* use.

The invention is further illustrated by the following examples, however, without being limited to the example or by any specific embodiment of the examples.

**Examples of the invention**

**Example 1: Comparing aldo-keto reductases capable of transforming Type B trichothecenes (e.g., DON to epi-THS)**

Three aldo-keto reductases Akr I, Akr II and Akr III (SEQ ID NO: 1, SEQ ID NO: 2, SEQ ID NO: 3, respectively), were aligned to one another applying the BLASTP algorithm (Altschul et al. 1990. J Mol Biol 215(3): 403-410) for aligning two sequences using default settings, *i.e.* Expect threshold: 10; Word size: 3; Max matches in a query range: 0; Matrix: BLOSUM62; Gap Costs: Existence: 11, Extension: 1; Compositional adjustments: Conditional compositional score matrix adjustment; No filters, no masking. The obtained values for sequence identities in percent are shown in Table 1. The Akrs were found to comprise a catalytic tetrade consisting of D54, Y59, K84 and H125, see Figure 1.

Table 1: Sequence identities in % determined with BLASTP with default settings (Expect threshold: 10; Word size: 3; Max matches in a query range: 0; Matrix: BLOSUM62; Gap Costs: Existence: 11, Extension: 1; Compositional adjustments: Conditional compositional score matrix adjustment; No filters, no masking).

| | Akr I (SEQ ID NO: 1) | Akr II (SEQ ID NO: 2) | Akr III (SEQ ID NO: 3) |
|---|---|---|---|
| Akr I (SEQ ID NO: 1) | 100 | 94 | 94 |
| Akr II (SEQ ID NO: 2) | | 100 | 99 |
| Akr III (SEQ ID NO: 3) | | | 100 |

By applying the assay described in Example 3, all three Akrs were found to catalyze the biotransformation of DON at the C8 atom to 3,7,8,15-tetrahydroxyscirpene (epi-THS) while oxidizing NADPH to $NADP^+$, as shown in Fig. 7a. No reverse reaction of epi-THS back to DON was observed.

**Example 2: Production of recombinant enzyme preparations**

For expression and protein purification of all candidate enzymes, nucleotide sequences encoding for the polypeptides of enzymes SEQ ID NOs: 1 - 5 were integrated into defined plasmids for expression in a suitable host organism, such as *Escherichia coli DH10B.* The recombinant vectors containing the respective coding sequences were assembled according to the standard procedures described in the respective manuals.

Nucleotide sequences encoding for SEQ ID NO. 1 - 3 (Akr I-III), SEQ ID NO. 4 (ADH-Lk), and SEQ ID NO: 5 (TAM-Ac) were amplified and sub-cloned into the commercial expression vectors pET3a or related vectors with restriction sites *NdeI* at the 5'end and *BamHI* or *XhoI* at the 3'end, and a 6xHis Tag at the C-terminal end, or expression vector pASK-IBA5Plus with restriction sites *BsaI* (*Eco31I*) at the 5' and at the 3'end, and a StrepTag_at the N-terminal end.

For production of enzymes with SEQ ID NOs: 1 - 5, the respective recombinant vectors were transferred into *E. coli* BL21(DE3), or any other suitable *E. coli* expression strain. Protein expression in cell cultures was induced with either IPTG (Isopropyl-β-D-thiogalactopyranosid) or AHTC (anhydrotetracycline). After culturing the cells over night with aeration, cells were harvested by centrifugation, resuspended in KPi buffer (50 mM pH 7.0.5), Tris buffer (100 mM pH8, 150 mM NaCl, 1 mM EDTA), or any other comparable lysis buffer. Cells were broken open by ultrasound, French Press or any other suitable method to open up bacterial cells and produce crude cell lysates. Cell lysates were cleared from cell debris via centrifugation and the resulting cleared lysate was subjected to affinity chromatography employing Nickel-sepharose or any other matrix of choice, to selectively purify the recombinant enzyme from the cleared lysate.

To determine the purity of the recombinant enzymes, they were separated on an SDS PAGE protein gel to visualize the proteins with standard Coomassie Blue Staining. The concentrations of the different proteins were determined photometrically in a plated reader (Biotek, Synergy HT) with a wave length of 595 nm using the Bradford reagent (Sigma # B6916) and/or with fluorimetric measurements using the Qubit protein assay (Thermofischer Life technologies #Q33211). For the Bradford assay, a standard curve that measured Bovine Serum Albumin (BSA, Sigma #A4919) in concentrations from 0 μg ml-1 to maximum 1500 μg ml-1, built the base for protein concentration measurements. For the Qubit analysis, the standard curve was established by measuring the provided standards from 0 μg ml-1 to 400 μg ml -1 and subsequently used to determine protein concentration of the samples.

**Example 3: DON transformation assay with Akr I-III**

To determine the transformation of DON to epi-THS by Akr I-III, described herein, the following assay was performed. An assay buffer was prepared containing 40 ppm DON, 1 mM NADPH in Teorell Stenhagen (TS) buffer, pH 6.0 (Ostling and Virtama, Acta Physiologica Scandinavica, 1946, 11:4, 289-293). Tubes containing the assay buffer were transferred in a heating block at 30 °C. The reaction was started by addition of Akr I-III to a final concentration of 200 nM in a final reaction volume of 200 μL. Samples were taken before reaction start, and 2, 5, 10, 15 and 20 min after reaction start. DON transformation reactions in these samples were stopped by mixing with absolute methanol at a 1:2 ratio, followed by a 1:100 dilution with 40% methanol. The concentrations of DON and epi-THS in the samples were determined with liquid chromatography-mass spectrometry (LC-MS) on a Sciex 5500 triple quadrupole mass spectrometer coupled to Agilent 1290 series UHPLC system, using the following MS parameters: Acquisition duration: 4 min; Scan Type: MRM (MRM); Polarity: Negative; Ion Source: Turbo Spray. Analyte parameters are shown in Table 2.

**Table 2:** MS analyte parameters. Q1 and Q3 refer to qualifier ions that enable identification of the target molecules, when they are present at the correct amount relative to the target ion characteristic for a given compound.

| Analyte | Q1 mass | Q3 mass |
|---|---|---|
| DON | 355.086 | 59.0 |
| DON QL | 355.086 | 265.0 |
| epi-THS | 357.058 | 297.2 |
| epi-THS QL | 357.058 | 177.1 |

For LC, eluent A was 5% methanol, 94.9% water (ultrapure), 0.1% acetic acid, and eluent B was 99.9% methanol, 0.1% acetic acid, using a Phenomenex Kinetex 2.6 μm biphenyl 150 x 2.1 mm column, an injection volume of 1 μL and the protocol shown in Table 3.

**Table 3:** LC eluent mix protocol.

| Time min | Flow rate μL/min | Eluent A % | Eluent B % |
|---|---|---|---|
| 0.00 | 500 | 95 | 5 |
| 0.10 | 500 | 95 | 5 |
| 3.00 | 500 | 30 | 70 |
| 3.01 | 500 | 0 | 100 |
| 3.30 | 500 | 0 | 100 |
| 3.31 | 500 | 95 | 5 |

Concentrations of DON and epi-THS throughout the time course of a DON transformation assay as described above are exemplarily shown in Figure 2.

**Example 4: Characterization of Akr I-III**

For further characterization of Akr I-III, DON transformation assays were performed at different pH values and at different temperatures, and enzyme stabilities were tested at different pH values and towards different temperatures.

By applying the DON transformation assay basically as described in Example 3, but at pH values ranging from pH 2 to pH 10, the pH optima for DON degradation of Akr I-III (SEQ ID NOs: 1 - 3) were determined. Akr I and Akr II reached maximum activities at pH 6.0, Akr III at pH 6.5. Akr I-III showed 50% activity or more of their respective maximum activity at pH values 4.5, 5.0 up to pH 9.0 and pH 9.5. For Akr I at pH 4.0 the reaction proceeded slower but complete degradation DON was still achieved after 30 min.

By applying the DON transformation assay basically as described in Example 3, but at different temperatures ranging from 14.9 °C to 55.7 °C, the temperature optima for the DON degradation activities of Akr I-III were determined. Akr I maximum activity was shown at approximately 35 °C, Akr II at approximately 31 °C, and Akr III at temperatures from approximately 28 °C to 33 °C (see Figure 4). Akr I-III showed more than 50% of its maximum activity at temperatures from approximately 20 °C to 48 °C. For Akr I even at temperatures below 20 °C (like 14.9 °C), complete DON degradation could be achieved after 20 min of incubation.

Kinetic parameters were determined by performing the DON transformation assay as described in Example 3. Akr I-III were used at a final concentration of 200 nM in Teorell Stenhagen (TS) buffer, pH 6.0 (Ostling and Virtama, Acta Physiologica Scandinavica, 1946, 11:4, 289-293)further containing 1 mM NADPH and DON at a concentration of either 3.38 μM, 33.78 μM, 135.14 μM, 270.27 μM or 1182.43 μM. The observed reaction rates and the corresponding DON concentrations were fitted to the Michaelis-Menten function used to describe enzyme kinetics. The fitting was done using the program SigmaPlot (Systat Software Inc.) to calculate the maximum DON transformation rate of Akr I-III in the assay (Vmax), the Michaelis constant ($K_M$) and the turnover number ($k_{cat}$). For Akr I, a Vmax of 8471 U/mg, a $K_M$ of 143 μM and a $k_{cat}$ of 5.08 /s were determined. Figure 5 shows an example of a non-linear curve fit.

The storage stability of Akr I-III at different pH values was tested by incubating 1 μM enzyme in TS buffer solutions with a pH of 3.0 - 10.0 for 4 h at 30°C in standard 1.5 ml Eppendorf tubes. Every hour, a DON transformation assay was conducted as described in Example 3. The starting activity at time 0 min was set to 100% and the relative residual activities were determined throughout the incubation. For the DON transformation assays, 80 μl of the storage solution were added to an assay reaction with a final volume of 400 μl, which corresponds to a 1:5 dilution of the storage solution. Akr I retained more than 93% activity during 4 hours at 30°C from pH 4.0 - 10.0. Akr II retained 30% to 93% activity during 4 hours at 30°C from pH 4.0 - 9.0. Akr III retained 27% to 85% activity during 4 hours at 30°C from pH 4.0 - 9.0. Akr I-III retained more than 40% of their activity for 4 hours at 30°C from pH 4.0 to pH 8.0. Akr I-III retained more than 55% of their activity for 4 hours at 30°C from pH 4.0 to pH 7.0, as displayed in Tables 4, 5 and 6.

**Table 4:** Relative DON-transformation activities of Akr I during 4 h of storage at 30 °C at different pH values.

| Time h | pH 3.0 | pH 4.0 | pH 5.0 | pH 6.0 | pH 7.0.0 | pH 8.0 | pH 9.0.0 | pH 10.0 |
|---|---|---|---|---|---|---|---|---|
| **0** | 100.0 | 100.0 | 100.0 | 100.0 | 100.0 | 100.0 | 100.0 | 100.0 |
| **1** | 0.0 | 100.6 | 101.0 | 100.6 | 99.6 | 101.5 | 100.9 | 100.5 |
| **2** | 0.0 | 102.8 | 101.6 | 101.9 | 101.4 | 102.4 | 101.0 | 101.4 |
| **3** | 0.0 | 101.5 | 100.8 | 101.1 | 99.7 | 101.1 | 100.8 | 99.8 |
| **4** | 0.0 | 93.8 | 97.9 | 100.7 | 96.1 | 101.2 | 96.6 | 96.0 |

**Table 5:** Relative DON-transformation activities of Akr II during 4 h of storage at 30 °C at different pH values.

| Time h | pH 3.0 | pH 4.0 | pH 5.0 | pH 6.0 | pH 7.0.0 | pH 8.0 | pH 9.0.0 | pH 10.0 |
|---|---|---|---|---|---|---|---|---|
| **0** | 100.0 | 100.0 | 100.0 | 100.0 | 100.0 | 100.0 | 100.0 | 100.0 |
| **1** | 34.5 | 99.3 | 100.1 | 77.3 | 79.6 | 85.1 | 76.5 | 54.4 |
| **2** | 1.1 | 96.6 | 99.8 | 85.1 | 64.5 | 68.3 | 54.7 | 37.7 |
| **3** | 0.0 | 87.1 | 99.3 | 77.6 | 56.9 | 53.2 | 36.1 | 13.9 |
| **4** | 0.0 | 89.0 | 92.4 | 61.8 | 57.4 | 40.9 | 29.9 | 7.2 |

**Table 6:** Relative DON-transformation activities of Akr III during 4 h of storage at 30 °C at different pH values.

| Time h | pH 3.0 | pH 4.0 | pH 5.0 | pH 6.0 | pH 7.0.0 | pH 8.0 | pH 9.0.0 | pH 10.0 |
|---|---|---|---|---|---|---|---|---|
| **0** | 100.0 | 100.0 | 100.0 | 100.0 | 100.0 | 100.0 | 100.0 | 100.0 |
| **1** | 13.5 | 95.1 | 97.7 | 97.8 | 93.0 | 94.2 | 76.3 | 47.0 |
| **2** | 19.3 | 84.4 | 96.3 | 94.9 | 91.3 | 77.9 | 61.3 | 27.1 |
| **3** | 0.0 | 74.8 | 90.7 | 93.1 | 85.1 | 69.0 | 54.1 | 17.0 |
| **4** | 0.0 | 57.7 | 85.2 | 78.9 | 74.0 | 57.6 | 27.4 | 15.4 |

The storage stability of Akr I-III was tested by incubation of 5 μM enzyme solutions in storage buffer (50 mM Tris pH 9.0, 0.5M NaCl, 50% Glycerol), for 60 min in a thermocycler (Mastercycler, Eppendorf) set to gradient mode from 30 °C to 50 °C. Throughout the incubation, samples were removed and placed on ice until DON transformation assays were performed as described in Example 3 to determine enzyme activities. The starting activity at time 0 min was set to 100% and the relative residual activities were determined throughout the incubation. Akr I retained more than 33% activity during 60 min in pH 6 at temperatures from 29.9°C to 46.0°C. Akr II retained 15% to 90% activity during 20 min in pH 6 at temperatures from 29.9°C to 40.7°C. Akr III retained more than 50% activity during 60 min in pH 6 at temperatures from 29.9°C to 46.0°C as displayed in Tables 7, 8 and 9.

Table 7: Relative DON transformation activities of Akr I during 60 min of incubation in storage buffer at different temperatures.

| Time, min | 29.9 °C | 35.3 °C | 37.9 °C | 40.7 °C | 43.4 °C | 46.0 °C | 50.8 °C |
|---|---|---|---|---|---|---|---|
| 0 | 100.00 | 100.00 | 100.00 | 100.00 | 100.00 | 100.00 | 100.00 |
| 5 | 121.5 | 132.8 | 130.1 | 130.2 | 133.6 | 125.9 | 118.6 |
| 10 | 114.8 | 97.7 | 101.4 | 100.7 | 111.3 | 123.1 | 44.2 |
| 20 | 118.9 | 101.5 | 110.1 | 60.5 | 79.1 | 83.4 | 16.3 |
| 35 | 106.2 | 109.7 | 109.0 | 95.8 | 75.2 | 61.8 | 4.9 |
| 50 | 70.1 | 84.6 | 77.0 | 76.1 | 60.2 | 3.0 | 7.5 |
| 60 | 53.7 | 47.0 | 48.6 | 50.0 | 52.9 | 33.1 | 0 |

**Table 8:** Relative DON transformation activities of Akr II during 60 min of incubation in storage buffer at different temperatures.

| Time, min | 29.9 °C | 35.3 °C | 37.9 °C | 40.7 °C | 43.4 °C | 46.0 °C | 50.8 °C |
|---|---|---|---|---|---|---|---|
| 0 | 100.00 | 100.00 | 100.00 | 100.00 | 100.00 | 100.00 | 100.00 |
| 5 | 95.8 | 91.9 | 90.5 | 61.5 | 39.2 | 2.9 | 4.7 |
| 10 | 91.6 | 92.5 | 80.2 | 56.1 | 34.9 | 4.7 | 5.7 |
| 20 | 90.3 | 86.0 | 65.0 | 15.5 | 21.9 | 0.00 | 9.4 |
| 35 | 89.4 | 77.4 | 38.1 | 11.3 | 7.1 | 2.7 | 2.4 |
| 50 | 84.1 | 49.6 | 10.6 | 0.00 | 0.00 | 0.00 | 0.00 |
| 60 | 86.8 | 46.4 | 6.2 | 0.00 | 0.00 | 0.00 | 0.00 |

**Table 9:** Relative DON transformation activities of Akr III during 60 min of incubation in storage buffer at different temperatures.

| Time, min | 29.9 °C | 35.3 °C | 37.9 °C | 40.7 °C | 43.4 °C | 46.0 °C | 50.8 °C |
|---|---|---|---|---|---|---|---|
| 0 | 100.00 | 100.00 | 100.00 | 100.00 | 100.00 | 100.00 | 100.00 |
| 5 | 89.1 | 96.00 | 94.01 | 104.1 | 83.4 | 97.7 | 77.4 |
| 10 | 91.6 | 65.2 | 74.5 | 76.1 | 68.1 | 62.6 | 72.7 |
| 20 | 90.3 | 98.6 | 43.9 | 81.6 | 66.7 | 62.5 | 19.5 |
| 35 | 93.1 | 75.2 | 75.2 | 104.5 | 89.3 | 58.4 | 16.6 |
| 50 | 74.0 | 80.2 | 80.9 | 75.8 | 81.3 | 23.3 | 10.9 |
| 60 | 72.2 | 104.3 | 78.6 | 105.1 | 91.8 | 50.4 | 16.0 |

**Example 5: Akr activity in complex matrices**

The DON transformation activities of Akr I-III were tested in complex matrices to verify the applicability of the enzymes as feed additive(s). As a feed matrix, piglet feed (FAF1, Waxenecker KG) was used. Piglet feed is composed of 10% wheat and 90% KON (Königshofer AG). Akr I, Akr II, or Akr III was mixed with 300 mg piglet feed and NADPH to achieve a final concentration of 200 nM enzyme, a final concentration of 1 mM NADPH and a final concentration of 40 ppm DON in a final volume of 3 mL deionized water. After 15 min, Akr I-III transformed DON to a concentration lower than the detection level.

As yet another complex matrix, untreated as well as heat-treated (boiled at 99°C for 15 min) rumen fluid were tested. Again, DON transformation was tested using 200 nM of either Akr I-III, 40 ppm DON and 1 mM NADPH. Akr I-III were found active and converted DON to epi-THS. In untreated rumen fluid, approximately 50% of DON was transformed after 15 min and more than 95% of DON was transformed after 15 min in heat-treated rumen fluid.

**Example 6: DON transformation assay with ADH-Lk**

For reduction of DON to 3,7,8R,15-tetrahydroxyscirpine (THS) catalyzed by SEQ ID NO. 4 (ADH-Lk), a degradation assay was set up that consists of: 50 mM Tris buffer pH 7.0.5 containing 1 mM NADP+, 5% v/v isopropanol, IPA, 100 ppm DON, and 200 nM enzyme. All components of the reaction except of isopropanol and NADP+ were mixed on ice and a 0 minutes sample was removed (mix sample 1:1 (v/v) with 100% methanol to stop any reaction). Immediately thereafter, reaction mixtures were transferred to the heating block and the reaction was started by adding NADP+ and IPA. The reaction was allowed to proceed for 22 hours at 30°C. A control reaction with the same set up, but lacking the enzyme was set up in parallel. Samples were removed in regular intervals in order to monitor the progression of the reaction and immediately mixed 1:1 (v/v) with 100% methanol to stop the reaction. They were subsequently analyzed using the HPLC-MS/MS method described in below.

For HPLC-MS/MS analysis, DON and THS were separated on a 150 mm x 2.1 mm Phenomenex Kinetex Biphenyl column with a particle size of 2.6 μm. The mobile phase consisted of a mixture of methanol and ultrapure water with 0.1 % (v/v) acetic acid. Ions were generated using electro spray ionization (ESI) in negative ionization mode. The quantification is done using a QTrap and/or triple quadrupole mass detector. Samples need to be diluted to fall into the linear range of this method which ranges from analyte concentrations of 1 ppb to 500 ppb in the injected sample. Results are depicted in Table 10. Presence of ADH-Lk in the reaction leads to the production of THS from DON.

**Table 10.** DON conversion to THS with ADH-Lk after 3.5 hours and 22 hours at 30°C.

| Reaction type | Time (h) | DON (μg/L) | THS [peak area] |
|---|---|---|---|
| Control | 0 | 395.0 | nd |
| Control | 3.5 | 397.3 | nd |
| Control | 22 | 371.0 | nd |
| ADH-Lk | 0 | 399.9 | nd |
| ADH-Lk | 3.5 | 387.1 | 7.88E+03 |
| ADH-Lk | 22 | 296.6 | 1.13E+05 |

**Example 7: Metabolite toxicity tests**

To determine whether or not the conversion of DON into epi-THS through Akr I-III and the conversion of DON into THS through ADH-Lk, as described herein, leads to a decrease in toxicity, two assays were performed. The in vitro TNT® Quick Coupled Transcription/ Translation System (Promega) was used to measure how well the metabolites inhibit protein synthesis and the CellTox™ Green Cytotoxicity Assay (Promega; G8741) was used to compare the toxic effects of the metabolites on primary epithelial cells derived from rainbow trout.

For the *in vitro* transcription/ translation assay, 40 μl of the TNT T7 quick master mix were mixed with 2 μl firefly luciferase encoding DNA and with 4 μl metabolites to obtain final concentrations from 0.01 μM - 150 μM in the reaction. Reactions lacking metabolites were the reference for maximum luminescence output. Reactions were incubated at 30°C for 90 min to allow for transcription of luciferase gene and subsequent translation. In an opaque (white) 96-well plate (Greiner Bio One) 100 μl Luciferase reagent where mixed with 4 μl of the reactions and immediately placed into a Synergy HT plate reader (Biotek) to read luminescence with a 2 sec and a 10 sec measurement delay. The lower the luminescence output in comparison to the reference, the more toxic the analysed metabolite. Both metabolites, epi-THS and THS are about 25-30 times less toxic in this assay compared to DON, as shown in Figure 6.

For cytotoxicity, cells were seeded into opaque 96-well plates (Greiner Bio One) and grown to subconfluence. Metabolites of interest were diluted to 0 - 30μM in Leibovitz L15 medium supplemented with 10% fetal bovine serum (FBS) and added to the cells, followed by incubation at 21°C for 24 h. Then 100 μl celltox green dye was added to the cells and the plate was incubated in the dark for 15 min at room temperature. Fluorescence was measured on a Synergy HT plate reader (Biotek) using the following parameters: temperature 30°C, excitation at 485 nm, emission at 528 nm, and a gain set to 100. The higher the fluorescence output, the more cells are affected and the more toxic the tested compound.

The primary cell line showed that both epi-THS and THS are at least 10 times less toxic than DON. The estimated LC50 (concentration at which 50% of cells are dead) value is between 0 -1 μM for DON and at ~ 15 μM for epi-THS, and THS, see Table 10.

**Table 10.** Toxicity of DON, epi-THS, and THS towards epithelial cells. Fluorescence measured with the highest DON concentration was used as 100% toxicity and all other measurements were related to this value.

| [c]/metabolite | DON | Epi-THS | THS |
|---|---|---|---|
| **30 μM** | 100 | 87.4 | 72.4 |
| **15 μM** | 87.8 | 58.1 | 42.8 |
| **10 μM** | 85.6 | 37.6 | 40.0 |
| **3 μM** | 64.2 | 38.5 | 33.5 |
| **1 μM** | 59.0 | 34.5 | 35.8 |

**Example 8: Transamination of DON**

The transamination of DON was studied using a transaminase. Transaminases (EC 2.6.1.-) catalyze the reversible transfer of an amino group from an amine to a carbonyl compound (acceptor). Our studies employed excess isopropylamine IPA as amine donor and as compound for the recycling of the cofactor pyridoxal-5'-phosphate (PLP). The excess of isopropylamine IPA has also the function of driving the unfavorable equilibrium towards the amine formation (Cassimjee et al, Chem. Commun. 2010, 46, 5569-5571.). For the regeneration of PLP, L-alanine Dehydrogenase (Koszelewski et al, Chem. Int. Ed. 2008, 47, 9337-9340) or the lactate dehydrogenase (Shin and Kim, Biotechnol. Bioeng. 1999, 65, 206-211) recycling systems, are also suitable as not all the transaminases accept the co-substrate isopropylamine (especially in the high concentrations required for the driving force of the equilibrium).

For amination of DON and its derivatives with additional carbonyl groups, such as 3-keto DON- to aminated metabolites catalyzed by SEQ ID NO. 5 (TAM-Ac), a transformation assay was set up that consists of: 100 mM Isopropylamine/$H_3PO_4$ pH 8 containing 1 mM pyridoxal 5'phosphate monohydrate (PLP), 200 ppm DON or 200 ppm DON derivative, and 200 nM enzyme. All components of the reaction were mixed on ice and a 0 minutes sample was removed and mixed 1:1 (v/v) with 60% MeOH/KPi pH 8. Immediately thereafter, reaction mixtures were transferred to the heating block and the reaction was allowed to proceed for 24 hours at 30°C. The control reaction had the same set up, but lacked the enzyme. Samples were removed in regular intervals to monitor the progression of the reaction. Samples were immediately mixed 1:1 (v/v) with 60% MeOH/KPi pH 8 to stop the reaction. They were subsequently analyzed using the QTOF-MS method described in below.

For QTOF-MS analysis (1290 infinity II LC combined with MS X500R QTOF), DON, 3-keto DON, 8-amino-DON, and 8-amino-3-keto DON were separated on a 100 mm x 2.1 mm Restek Raptor Biphenyl column with a particle size of 2.7 μm. The mobile phase consisted of a mixture of methanol, or acetonitrile and ultrapure water. Ions were generated using electro spray ionization (ESI) in positive ionization mode. The quantification is done using a TOF and/or triple quadrupole mass detector. Samples need to be diluted to fall into the linear range of this method which ranges from analyte concentrations of 1 ppb to 500 ppb in the injected sample. Results are depicted in Table 11. Presence of SEQ ID NO. 5 in the reaction leads to the production of aminated DON, and DON derivatives, such as 8-amino DON and 8-amino-3-keto DON.

Table 11. QTOF analysis results for DON and 3-keto DON. NC prefix refers to the negative control for the respective substrate lacking the enzyme.

| | **DON** | **8-amino-DON** | **3-keto-DON** | **8-amino-3-keto-DON** | **8-amino-3-keto-DON** |
|---|---|---|---|---|---|
| **Sample** | | | | RT 0.6 min | RT 1.2 min |
| **blank** | N/A | N/A | 2.51 E+03 | N/A | N/A |
| **DON T0h** | 1.29E+06 | 4.84E+03 | 2.34E+03 | 7.24E+01 | N/A |
| **DON T3h** | 1.26E+06 | 5.39E+03 | 3.29E+02 | N/A | N/A |
| **DON T18h** | 1.32E+06 | 4.73E+03 | 2.60E+03 | N/A | N/A |
| **3-keto-DON T0h** | 1.07E+03 | 6.47E+02 | 2.69E+05 | N/A | N/A |
| **3-keto-DON T3h** | 1.17E+03 | 5.56E+02 | 2.62E+05 | 8.32E+03 | 1.03E+04 |
| **3-keto-DON T18h** | 1.84E+03 | 2.70E+02 | 3.02E+05 | 1.04E+05 | 1.32E+05 |
| **NC DON T0h** | 1.22E+06 | 5.09E+03 | 2.74E+02 | N/A | N/A |
| **NC DON T3h** | 1.25E+06 | 4.89E+03 | 5.82E+02 | N/A | N/A |
| **NC DON T18h** | 1.38E+06 | 5.42E+03 | 2.25E+03 | N/A | N/A |
| **NC 3-keto-DON T0h** | 6.19E+02 | 7.44E+02 | 2.46E+05 | N/A | N/A |
| **NC 3-keto-DON** | 1.43E+03 | 8.59E+02 | 2.58E+05 | N/A | N/A |
| **T3h** | | | | | |
| **NC 3-keto-DON T18h** | 1.46E+03 | N/A | 2.61 E+05 | N/A | N/A |
| blank | N/A | N/A | 2.33E+03 | N/A | N/A |

To determine the toxicity of 8-amino DON, the in vitro transcription/ translation assay was performed as described in Example 8, with the difference that 8-amino-DON was compared to DON and had a 5 - 10 times weaker effect on luminescence output.

## Claims

1. A polypeptide capable of modifying the C8-atom of a Type B trichothecene, preferably DON or a DON derivative, wherein said polypeptide is one or more of the following:

   a) a polypeptide having at least 94.1% identity (e.g., at least 94.2%, at least 94.3%, at least 94.4% at least 94.5%, at least 94.6%, at least 94.7%, at least 94.8%, at least 94.9%, at least 95%, at least 96%, at least 97%, at least 98%, at least 99% or 100%) to the polypeptide set forth in SEQ ID NO: 2 or SEQ ID NO: 3;
   b) a polypeptide having at least 91.1% identity (e.g., at least 91.2%, at least 91.3%, at least 91.4% at least 91.5%, at least 91.6%, at least 91.7%, at least 91.8%, at least 91.9%, at least 92%, at least 93%, at least 94%, at least 95%, at least 96%, at least 97%, at least 98%, at least 99% or 100%) to the polypeptide set forth in SEQ ID NO: 4;
   c) a polypeptide having at least 70% sequence identity (e.g., at least 71%, at least 72%, at least 73%, at least 74%, at least 75%, at least 76%, at least 77%, at least 78%, at least 79%, at least 80%, at least 81%, at least 82%, at least 83%, at least 84%, at least 85%, at least 86%, at least 87%, at least 88%, at least 89%, at least 90%, at least 91%, at least 92%, at least 93%, at least 94%, at least 95%, at least 96%, at least 97%, at least 98%, at least 99% or 100%) to the polypeptide of: SEQ ID NO: 1, SEQ ID NO: 2, SEQ ID NO: 3, SEQ ID NO: 4 or SEQ ID NO: 5;
   d) a variant of the polypeptide of SEQ ID NO: 1, SEQ ID NO: 2, SEQ ID NO: 3, SEQ ID NO: 4 or SEQ ID NO: 5, wherein said variant comprising a substitution, deletion, and/or insertion at one or more positions, wherein said variant having at least 70% (e.g., at least 71 %, at least 72%, at least 73%, at least 74%, at least 75%, at least 76%, at least 77%, at least 78%, at least 79%, at least 80%, at least 81 %, at least 82%, at least 83%, at least 84%, at least 85%, at least 86%, at least 87%, at least 88%, at least 89%, at least 90%, at least 91 %, at least 92%, at least 93%, at least 94%, at least 95%, at least 95.5%, at least 96%, at least 96.5%, at least 97%, at least 97.5%, at least 98%, at least 98.5%, at least 99%), but less than 100% sequence identity with the amino acid sequence set forth in SEQ ID NO: 1, SEQ ID NO: 2, SEQ ID NO: 3, SEQ ID NO: 4 or SEQ ID NO: 5;
   e) a fragment of the polypeptide of (a)-(d) capable of modifying the C8-atom of the Type B trichothecene, preferably DON or a DON derivative.

2. The polypeptide according to any one of the preceding claims, wherein: (i) said modifying of the C8-atom is one or more of the following: reducing a keto moiety (preferably reducing said keto moiety to a hydroxyl moiety), transaminating a keto moiety, detoxifying and/or reducing the toxicity of the Type B trichothecene, preferably DON or a DON derivative; and/or (ii) said Type B trichothecene, preferably a DON derivative, is selected from the group consisting of: 3-Acetyldeoxynivalenol, 15-Acetyldeoxynivalenol, Nivalenol, 4-acetylnivalenol, 3-amino Deoxynivalenol.

3. The polypeptide according to any one of the preceding claims, wherein said modifying the C8-atom is a reduction and/or transamination of the C8-atom leading to one or more of the following: 3,7,8,15-tetrahydroxyscirpene (epi-THS), 3,7,8R,15-tetrahydroxyscirpine (THS), 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol.

4. A recombinant host cell (e.g., an isolated recombinant host cell), transgenic plant, transgenic seed or transgenic pollen grain comprising one or more of the following:

   i) one or more polypeptides according to any one of the preceding claims;
   ii) one or more polynucleotides encoding one or more polypeptides according to any one of the preceding claims;
   iii) one or more nucleic acid constructs and/or expression vectors capable of expressing one or more polynucleotides encoding one or more polypeptides according to any one of the preceding claims.

5. A foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic and/or mixture/s thereof, comprising one or more of the following: polypeptide/s, polynucleotide/s encoding one or more polypeptides according to any one of the preceding claims, nucleic acid construct/s capable of expressing one or more polynucleotides encoding one or more polypeptides according to any one of the preceding claims, expression vector/s capable of expressing one or more polynucleotides encoding one or more polypeptides according to any one of the preceding claims, recombinant host cell/s, transgenic plant/s, transgenic seed/s and/or transgenic pollen grain/s according to any one of the preceding claims.

6. A composition or kit comprising one or more of the following: polypeptide/s according to any one of the preceding claims, polynucleotide/s encoding one or more polypeptides according to any one of the preceding claims, nucleic acid construct/s capable of expressing one or more polynucleotides encoding one or more polypeptides according to any one of the preceding claims, expression vector/s capable of expressing one or more polynucleotides encoding one or more polypeptides according to any one of the preceding claims and/or recombinant host cell/s, transgenic plant/s, transgenic seed/s, transgenic pollen grain/s, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement, prebiotic, intermediate prebiotic and/or mixture/s thereof according to any one of the preceding claims.

7. A Type B trichothecene compound or intermediate modified at the C8-atom, said compound or intermediate is selected from the group consisting of: 3,7,8,15-tetrahydroxyscirpene (epi-THS), 3,7,8R,15-tetrahydroxyscirpine (THS), 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy-Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and 8-Amino-3-amino Deoxynivalenol.

8. A method for producing a Type B trichothecene compound or intermediate modified at the C8-atom, said compound or intermediate is selected from the group consisting of: 3,7,8,15-tetrahydroxyscirpene (epi-THS), 3,7,8R,15-tetrahydroxyscirpine (THS), 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy-Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and 8-Amino-3-amino Deoxynivalenol, said method comprising: applying one or more polypeptide/s, recombinant cell/s, composition/s and/or kit/s according to any one of the preceding claims to a corresponding Type B trichothecene, preferably DON or a DON derivative.

9. A Type B trichothecene compound or intermediate modified at the C8-atom, said compound or intermediate is selected from the group consisting of: 3,7,8,15-tetrahydroxyscirpene (epi-THS), 3,7,8R,15-tetrahydroxyscirpine (THS), 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy-Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and 8-Amino-3-amino Deoxynivalenol, produced by the method of producing the compound or intermediate according to any one of the preceding claims.

10. A method for one or more of the following, in a substrate or sample comprising a Type B trichothecene, preferably DON and/or DON derivative/s:

i) for modifying the C8-atom of a Type B trichothecene, preferably DON and/or a DON derivative/s;
ii) for altering the level of a Type B trichothecene, preferably DON and/or a DON derivative/s (e.g., reducing the level); and/or detoxifying a Type B trichothecene, preferably DON and/or DON derivative/s;
iii) for bio-transforming (e.g., enzymatically modifying) a Type B trichothecene, preferably DON and/or DON derivative/s, preferably said bio-transforming is enzymatic;
iv) for detecting and/or assessing the level of a Type B trichothecene mycotoxin, preferably DON and/or DON derivative/s; and/or

v) for reducing the toxicity of a Type B trichothecene, preferably DON and/or a DON derivative/s and/or for treating seeds;

said method comprising:

(a) providing one or more of the following: polypeptide/s, compound/s, intermediate/s, polynucleotide/s encoding one or more polypeptides according to any one of the preceding claims, nucleic acid construct/s capable of expressing one or more polynucleotides encoding one or more polypeptides according to any one of the preceding claims, expression vector/s capable of expressing one or more polynucleotides encoding one or more polypeptides according to any one of the preceding claims, recombinant host cell/s, transgenic plant/s, transgenic seed/s, transgenic pollen grain/s, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit according to any one of the preceding claims;
(b) applying (a) to said substrate or sample;
(c) preferably: detecting and/or quantifying one or more Type B trichothecene compound/s or intermediate/s according to any one of the preceding claims.

**11.** The method according to any one of the preceding claims, wherein: (i) said modifying the C8-atom comprises one or more of the following: reducing a keto moiety (preferably reducing said keto moiety to a hydroxyl moiety), transaminating a keto moiety, detoxifying and/or reducing the toxicity of the Type B trichothecene, preferably DON or a DON derivative/s; and/or (ii) said Type B trichothecene, preferably a DON derivative, is selected from the group consisting of: 3-Acetyldeoxynivalenol, 15-Acetyldeoxynivalenol, Nivalenol, 4-acetylnivalenol, 3-amino Deoxynivalenol.

**12.** A method for treatment, amelioration, prophylaxis and/or diagnostics of mycotoxicosis (e.g., in a subject or animal), comprising:

i) providing: a therapeutically efficient amount of one or more of the following: polypeptide/s, polynucleotide/s encoding one or more polypeptides according to any one of the preceding claims, nucleic acid construct/s capable of expressing one or more polynucleotides encoding one or more polypeptides according to any one of the preceding claims, expression vector/s capable of expressing one or more polynucleotides encoding one or more polypeptides according to any one of the preceding claims, recombinant host cell/s, composition/s or kit/s according to any one of the preceding claims;
ii) administering said therapeutically efficient amount as defined in (i) (e.g., to said subject or animal).

**13.** The polypeptide, compound, intermediate, polynucleotide encoding one or more polypeptides according to any one of the preceding claims, nucleic acid construct capable of expressing one or more polynucleotides encoding one or more polypeptides according to any one of the preceding claims, expression vector capable of expressing one or more polynucleotides encoding one or more polypeptides according to any one of the preceding claims, recombinant host cell, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit according to any one of the preceding claims, for use in therapy, prophylaxis and/or as a medicament (e.g., for veterinary use).

**14.** The polypeptide, compound or intermediate, polynucleotide encoding one or more polypeptides according to any one of the preceding claims, nucleic acid construct capable of expressing one or more polynucleotides encoding one or more polypeptides according to any one of the preceding claims, expression vector capable of expressing one or more polynucleotides encoding one or more polypeptides according to any one of the preceding claims, recombinant host cell, transgenic plant, transgenic seed, transgenic pollen grain, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit according to any one of the preceding claims, for use in one or more of the following methods:

i) method for modifying C8-atom of a Type B trichothecene, preferably DON and/or a DON derivative/s;

ii) method for detoxifying a Type B trichothecene, preferably DON and/or a DON derivative/s having a keto moiety (C=O) at the C8 atom;
iii) method for bio-transforming a Type B trichothecene, preferably DON and/or a DON derivative/s having a keto moiety (C=O) at the C8 atom, preferably said bio-transforming is enzymatic;
iv) method for treatment, amelioration, prophylaxis and/or diagnostics of the Type B trichothecene mycotoxicosis, preferably DON mycotoxicosis;
v) method for monitoring development of mycotoxicosis and/or assessing the efficacy of the Type B trichothecene mycotoxicosis, prophylaxis and/or therapy, preferably DON-mycotoxicosis, prophylaxis and/or therapy;
vi) method for screening a candidate compound for the Type B trichothecene mycotoxicosis detoxification activity, preferably DON detoxification activity;
vii) method for detecting and/or assessing the level of the Type B trichothecene, preferably DON and/or a DON derivative/s;
viii) method for reducing the toxicity of the Type B trichothecene, preferably DON and/or a DON derivative/s;
ix) method for altering, preferably reducing, the level of the Type B trichothecene, preferably DON and/or a DON derivative/s
x) producing one or more of the following: foodstuff, intermediate foodstuff; fodder, intermediate fodder; feed intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic; pharmaceutical, veterinary, diagnostic, detoxifying, monitoring and/or screening composition or kit;
xi) method according to any one of the preceding claims;
xii) any combination of methods according to (i)-(xi);
xiii) method of any of (i)-(xii), wherein said method is an *in vitro, ex vivo* or *in vivo* method.

**15.** Use of one or more polypeptide/s, compound/s, intermediate/s, polynucleotide/s encoding one or more polypeptides according to any one of the preceding claims, nucleic acid construct/s capable of expressing one or more polynucleotides encoding one or more polypeptides according to any one of the preceding claims, expression vector/s capable of expressing one or more polynucleotides encoding one or more polypeptides according to any one of the preceding claims, recombinant host cell/s, transgenic plant/s, transgenic seed/s, transgenic pollen grain/s, foodstuff, intermediate foodstuff; fodder, intermediate fodder; feed, intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic or mixture/s thereof, composition or kit according to any one of the preceding claims for/in one or more of the following:

i) for modifying the C8-atom of a Type B trichothecene, preferably DON and/or DON derivative/s;
ii) for detoxifying a Type B trichothecene, preferably DON and/or DON derivative/s;
iii) for bio-transforming of a Type B trichothecene, preferably DON and/or DON derivative/s, having a keto moiety (C=O) at the C8-atom, preferably said bio-transforming is enzymatic;
iv) for treatment, amelioration, prophylaxis and/or diagnostics of a Type B trichothecene mycotoxicosis, preferably DON-mycotoxicosis;
v) for monitoring development of mycotoxicosis and/or assessing the efficacy of a Type B trichothecene mycotoxicosis, prophylaxis and/or therapy, preferably DON-mycotoxicosis prophylaxis and/or therapy;
vi) for screening a candidate compound for anti-mycotoxicosis activity;
vii) for detecting and/or assessing the level of a Type B trichothecene, preferably DON and/or DON derivative/s;
viii) for inhibiting toxicity of a Type B trichothecene, preferably DON and/or DON derivative/s;
ix) for altering, preferably reducing, the level of a Type B trichothecene, preferably DON and/or DON derivative/s;
x) for producing one or more of the following: a foodstuff, intermediate foodstuff; fodder, an intermediate fodder; feed, intermediate feed; additive (e.g., foodstuff-, fodder- or feed additive), intermediate additive (e.g., foodstuff-, fodder- or feed intermediate additive); detoxifying agent, an intermediate detoxifying agent; nutritional supplement, an intermediate nutritional supplement; prebiotic, intermediate prebiotic; and/or pharmaceutical- diagnostic-, detoxifying-, monitoring- and/or screening composition or kit.
xi) in the method according to any one of the preceding claims;
xii) any combination of (i)-(xi);
xiii) use according to any one (i)-(xii), wherein said use is an *in vitro, ex vivo* or *in vivo* use.

**Amended claims in accordance with Rule 137(2) EPC.**

1. A foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic and/or mixture/s thereof, comprising a polypeptide capable of modifying the C8-atom of a Type B trichothecene, preferably DON or a DON derivative, wherein said polypeptide is one or more of the following:

   a) a polypeptide having at least 94.1% identity to the polypeptide set forth in SEQ ID NO: 2 or SEQ ID NO: 3;
   b) a polypeptide having at least 91.1% identity to the polypeptide set forth in SEQ ID NO: 4;
   c) a polypeptide having at least 70% sequence identity to the polypeptide of: SEQ ID NO: 1, SEQ ID NO: 2, SEQ ID NO: 3, SEQ ID NO: 4 or SEQ ID NO: 5;
   d) a variant of the polypeptide of SEQ ID NO: 1, SEQ ID NO: 2, SEQ ID NO: 3, SEQ ID NO: 4 or SEQ ID NO: 5, wherein said variant comprising a substitution, deletion, and/or insertion at one or more positions, wherein said variant having at least 70%, but less than 100% sequence identity with the amino acid sequence set forth in SEQ ID NO: 1, SEQ ID NO: 2, SEQ ID NO: 3, SEQ ID NO: 4 or SEQ ID NO: 5;
   e) a fragment of the polypeptide of (a)-(d) capable of modifying the C8-atom of the Type B trichothecene, preferably DON or a DON derivative.

2. The foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic and/or mixture/s thereof according to claim 1, wherein: (i) said modifying of the C8-atom is one or more of the following: reducing a keto moiety (preferably reducing said keto moiety to a hydroxyl moiety), transaminating a keto moiety, detoxifying and/or reducing the toxicity of the Type B trichothecene, preferably DON or a DON derivative; and/or (ii) said Type B trichothecene, preferably a DON derivative, is selected from the group consisting of: 3-Acetyldeoxynivalenol, 15-Acetyldeoxynivalenol, Nivalenol, 4-acetylnivalenol, 3-amino Deoxynivalenol.

3. The foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic and/or mixture/s thereof according to any one of the preceding claims, wherein said modifying the C8-atom is a reduction and/or transamination of the C8-atom leading to one or more of the following: 3,7,8,15-tetrahydroxyscirpene (epi-THS), 3,7,8R,15-tetrahydroxyscirpine (THS), 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and/or 8-Amino-3-amino Deoxynivalenol.

4. The foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic and/or mixture/s thereof according to any one of the preceding claims, wherein said polypeptide has dehydrogenase and/or transaminase activity.

5. The foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic and/or mixture/s thereof according to any one of the preceding claims, wherein said polypeptide is stable within pH range from 5.0 to about 7.0, preferably from 4.0 to about 7.0.

6. The foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic and/or mixture/s thereof according to any one of the preceding claims, wherein said polypeptide is stable within a temperature range from about 30 °C to about 40 °C.

7. A foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic and/or mixture/s thereof comprising a recombinant host cell, transgenic plant, transgenic seed or transgenic pollen grain comprising one or more of the following:

i) one or more polypeptides as defined in any one of the preceding claims;
ii) one or more polynucleotides encoding one or more polypeptides as defined in any one of the preceding claims;
iii) one or more nucleic acid constructs and/or expression vectors capable of expressing one or more polynucleotides encoding one or more polypeptides as defined in any one of the preceding claims.

**8.** A foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic and/or mixture/s thereof, comprising one or more of the following:
polynucleotide/s encoding one or more polypeptides as defined in any one of the preceding claims, nucleic acid construct/s capable of expressing one or more polynucleotides encoding one or more polypeptides as defined in any one of the preceding claims, expression vector/s capable of expressing one or more polynucleotides encoding one or more polypeptides as defined in any one of the preceding claims, recombinant host cell/s, transgenic plant/s, transgenic seed/s and/or transgenic pollen grain/s as defined in any one of the preceding claims.

**9.** A composition or kit comprising one or more of the following:
polypeptide/s as defined in any one of the preceding claims, polynucleotide/s encoding one or more polypeptides as defined in any one of the preceding claims, nucleic acid construct/s capable of expressing one or more polynucleotides encoding one or more polypeptides as defined in any one of the preceding claims, expression vector/s capable of expressing one or more polynucleotides encoding one or more polypeptides as defined in any one of the preceding claims and/or recombinant host cell/s, transgenic plant/s, transgenic seed/s, transgenic pollen grain/s as defined in any one of the preceding claims, foodstuff, intermediate foodstuff; fodder, intermediate fodder; feed, intermediate feed; additive, intermediate additive; detoxifying agent, intermediate detoxifying agent; nutritional supplement, intermediate nutritional supplement, prebiotic, intermediate prebiotic and/or mixture/s thereof according to any one of the preceding claims.

**10.** A method for producing a Type B trichothecene compound or intermediate modified at the C8-atom, said compound or intermediate is selected from the group consisting of: 3,7,8,15-tetrahydroxyscirpene (epi-THS), 3,7,8R,15-tetrahydroxyscirpine (THS), 8-amino-DON, 8-amino-3-keto-DON, 8-Hydroxy-3-keto Deoxynivalenol, 8-Hydroxy-3-epi Deoxynivalenol, 8-Hydroxy-3-Acetyldeoxynivalenol, 8-Hydroxy-15-Acetyldeoxynivalenol, 8-Hydroxy- Nivalenol, 8-Hydroxy-Fusarenon-X, 8-Hydroxy-3-amino Deoxynivalenol, 8-Amino-3-epi Deoxynivalenol, 8-Amino-3-Acetyldeoxynivalenol, 8-Amino-15-Acetyldeoxynivalenol, 8-Amino-Nivalenol, 8-Amino-Fusarenon-X and 8-Amino-3-amino Deoxynivalenol, said method comprising: applying one or more polypeptide/s or recombinant cell/s as defined in any one of the preceding caims, composition/s and/or kit/s according to claim 9 to a corresponding Type B trichothecene, preferably DON or a DON derivative.

**11.** A method for one or more of the following, in a substrate or sample comprising a Type B trichothecene, preferably DON and/or DON derivative/s:

i) for modifying the C8-atom of a Type B trichothecene, preferably DON and/or a DON derivative/s;
ii) for altering the level of a Type B trichothecene, preferably DON and/or a DON derivative/s (e.g., reducing the level); and/or detoxifying a Type B trichothecene, preferably DON and/or DON derivative/s;
iii) for bio-transforming (e.g., enzymatically modifying) a Type B trichothecene, preferably DON and/or DON derivative/s, preferably said bio-transforming is enzymatic;
iv) for detecting and/or assessing the level of a Type B trichothecene mycotoxin, preferably DON and/or DON derivative/s; and/or
v) for reducing the toxicity of a Type B trichothecene, preferably DON and/or a DON derivative/s and/or for treating seeds;

said method comprising:

(a) providing one or more of the following: polypeptide/s or polynucleotide/s encoding one or more polypeptides as defined in any one of the preceding claims, nucleic acid construct/s capable of expressing one or more polynucleotides encoding one or more polypeptides as defined in any one of the preceding claims, expression vector/s capable of expressing one or more polynucleotides encoding one or more polypeptides as defined in any one of the preceding claims, recombinant host cell/s, transgenic plant/s, transgenic seed/s, transgenic pollen grain/s as defined in any one of the preceding claims, foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic or mixture/s thereof

according to any one of claims 1 to 8, composition or kit according to claim 9;
(b) applying (a) to said substrate or sample;
(c) preferably: detecting and/or quantifying one or more Type B trichothecene compound/s or intermediate/s according to any one of the preceding claims.

**12.** The method according to claim 8, wherein: (i) said modifying the C8-atom comprises one or more of the following: reducing a keto moiety (preferably reducing said keto moiety to a hydroxyl moiety), transaminating a keto moiety, detoxifying and/or reducing the toxicity of the Type B trichothecene, preferably DON or a DON derivative/s; and/or (ii) said Type B trichothecene, preferably a DON derivative, is selected from the group consisting of: 3-Acetyldeoxynivalenol, 15-Acetyldeoxynivalenol, Nivalenol, 4-acetylnivalenol, 3-amino Deoxynivalenol.

**13.** The polypeptide as defined in any one of the preceding claims, polynucleotide encoding one or more polypeptides as defined in any one of the preceding claims, nucleic acid construct capable of expressing one or more polynucleotides encoding one or more polypeptides as defined in any one of the preceding claims, expression vector capable of expressing one or more polynucleotides encoding one or more polypeptides as defined in any one of the preceding claims, recombinant host cell, transgenic plant, transgenic seed, transgenic pollen grain as defined in any one of the preceding claims, foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic or mixture/s thereof according to any one of claims 1 to 8, composition or kit according to claim 9, for use in therapy, prophylaxis and/or as a medicament.

**14.** The polypeptide as defined in any one of the preceding claims, polynucleotide encoding one or more polypeptides as defined in any one of the preceding claims, nucleic acid construct capable of expressing one or more polynucleotides encoding one or more polypeptides as defined in any one of the preceding claims, expression vector capable of expressing one or more polynucleotides encoding one or more polypeptides as defined in any one of the preceding claims, recombinant host cell, transgenic plant, transgenic seed, transgenic pollen grain as defined in any one of the preceding claims, foodstuff, intermediate foodstuff; fodder, intermediate fodder; feed, intermediate feed; additive (, intermediate additive; detoxifying agent, intermediate detoxifying agent; nutritional supplement, intermediate nutritional supplement; prebiotic, intermediate prebiotic or mixture/s thereof according to any one of claims 1 to 8, composition or kit according to claim 9, for use in one or more of the following methods:

i) method for modifying C8-atom of a Type B trichothecene, preferably DON and/or a DON derivative/s;
ii) method for detoxifying a Type B trichothecene, preferably DON and/or a DON derivative/s having a keto moiety (C=O) at the C8 atom;
iii) method for bio-transforming a Type B trichothecene, preferably DON and/or a DON derivative/s having a keto moiety (C=O) at the C8 atom, preferably said bio-transforming is enzymatic;
iv) method for treatment, amelioration, prophylaxis and/or diagnostics of the Type B trichothecene mycotoxicosis, preferably DON mycotoxicosis;
v) method for monitoring development of mycotoxicosis and/or assessing the efficacy of the Type B trichothecene mycotoxicosis, prophylaxis and/or therapy, preferably DON-mycotoxicosis, prophylaxis and/or therapy;
vi) method for screening a candidate compound for the Type B trichothecene mycotoxicosis detoxification activity, preferably DON detoxification activity;
vii) method for detecting and/or assessing the level of the Type B trichothecene, preferably DON and/or a DON derivative/s;
viii) method for reducing the toxicity of the Type B trichothecene, preferably DON and/or a DON derivative/s;
ix) method for altering, preferably reducing, the level of the Type B trichothecene, preferably DON and/or a DON derivative/s
x) producing one or more of the following: foodstuff, intermediate foodstuff; fodder, intermediate fodder; feed intermediate feed; additive (e.g., foodstuff-, fodder- or feed additive), intermediate additive (e.g., foodstuff-, fodder- or feed intermediate additive); detoxifying agent, intermediate detoxifying agent; nutritional supplement, intermediate nutritional supplement; prebiotic, intermediate prebiotic; pharmaceutical, veterinary, diagnostic, detoxifying, monitoring and/or screening composition or kit;
xi) method according to any one of the preceding claims;
xii) any combination of methods according to (i)-(xi);
xiii) method of any of (i)-(xii), wherein said method is an *in vitro, ex vivo* or *in vivo* method.

**15.** In vitro use of one or more polypeptide/s as defined in any one of the preceding claims, polynucleotide/s encoding one or more polypeptides as defined in any one of the preceding claims, nucleic acid construct/s capable of expressing

one or more polynucleotides encoding one or more polypeptides as defined in any one of the preceding claims, expression vector/s capable of expressing one or more polynucleotides encoding one or more polypeptides as defined in any one of the preceding claims, recombinant host cell/s, transgenic plant/s, transgenic seed/s, transgenic pollen grain/s as defined in any one of the preceding claims, foodstuff, intermediate foodstuff; fodder, intermediate fodder; feed, intermediate feed; additive, intermediate additive; detoxifying agent, intermediate detoxifying agent; nutritional supplement, intermediate nutritional supplement; prebiotic, intermediate prebiotic or mixture/s thereof according to any one of claims 1 to 8, composition or kit according to claim 9 for/in one or more of the following:

i) for modifying the C8-atom of a Type B trichothecene, preferably DON and/or DON derivative/s;
ii) for detoxifying a Type B trichothecene, preferably DON and/or DON derivative/s;
iii) for bio-transforming of a Type B trichothecene, preferably DON and/or DON derivative/s, having a keto moiety (C=O) at the C8-atom, preferably said bio-transforming is enzymatic;
iv) for screening a candidate compound for anti-mycotoxicosis activity;
v) for detecting and/or assessing the level of a Type B trichothecene, preferably DON and/or DON derivative/s;
vi) for inhibiting toxicity of a Type B trichothecene, preferably DON and/or DON derivative/s;
vii) for altering, preferably reducing, the level of a Type B trichothecene, preferably DON and/or DON derivative/s;
viii) for producing one or more of the following: a foodstuff, intermediate foodstuff; fodder, an intermediate fodder; feed, intermediate feed; additive, intermediate additive; detoxifying agent, an intermediate detoxifying agent; nutritional supplement, an intermediate nutritional supplement; prebiotic, intermediate prebiotic; and/or pharmaceutical- diagnostic-, detoxifying-, monitoring- and/or screening composition or kit.

FIG. 1

```
SEQ ID No.1   MRTTEISQGLKVSNLCIGAGPLGGMPTAFGYDVSEERGIATVQAVFDSPINFMDTSSEYG  60
SEQ ID No.2   MRTTEIAQGLKVSNLCIGAGPLGGMPTAFGYDVSEERGIATVQAVFDSPINFMDTSSEYG  60
SEQ ID No.3   MRTTEIAQGLKVSNLCIGAGPLGGMPTAFGYDVSEERGIATVQAVFDSPINFMDTSSEYG  60
              ******:*****************************************************

SEQ ID No.1   DSEVRIGKVIREGRMPKGFVIATKADAEGDGTARRGDFSAKQVRASFELSLERLGLTHID  120
SEQ ID No.2   GSEARIGKVIREGLMPKGFVISSKADAEGEGVGRRGDFSAKQVRASFELSLERLGLTHLD  120
SEQ ID No.3   GSEARIGKVIREGRMPKGFVISSKADAEGEGVGRRGDFSAKQVRASFELSLERLGLTHLD  120
              .**.********* *******::******:*..*************************:*

SEQ ID No.1   VYFLHDPEKFPFEHVMARGGAMEELLRLKDEGLVGLVGVAGGDVVEMARYVDTGNLDILL  180
SEQ ID No.2   VYFLHDPEKFPFEQVMARGGAMEELLRLKDEGLVGLVGVAGGDVLEMARYVDTGSLDILL  180
SEQ ID No.3   VYFLHDPEKFPFEQVMARGGAMEELLRLKDEGLVGLVGVAGGDVLEMARYVDTGSLDILL  180
              *************:******************************:*********.*****
```

FIG. 1 (CONT.)

```
SEQ ID No.1   NYGRYTLLDRSADSLIDKASDAGMAFLNAGPYASGLLAAPRGGSAWYQYAPPNADALQAV  240
SEQ ID No.2   NYGRYTLLDRSADSLITKVSDAGMAFLNAGPYASGLLAAPRGGSAWYQYAPPDATALQAV  240
SEQ ID No.3   NYGRYTILDRSADSLITKVSDAGMAFLNAGPYASGLLAAPRGGSAWYQYAPPDATALQAV  240
              ******:********* *.*********************************:* *****

SEQ ID No.1   DQLHELCESFDVSLRSVALQFSMRDPRITSTVVGLSRPERVRALLDDAAAEIPAELWDQL  300
SEQ ID No.2   DQLHELCESFDVSLRSVALQFSMRDPRITSTVVGLSRPERVRALLDDEAAEIPAELWEQL  300
SEQ ID No.3   DQLHELCESFDVSLRSVALQFSMRDPRITSTVVGLSRPERVRALLDDEAAEIPAELWEQL  300
              *********************************************** *********:**

SEQ ID No.1   PAPLNLPWNSLA 312
SEQ ID No.2   PAPLNGDIVLPG 312
SEQ ID No.3   PAPLNGDIVLPR 312
              *****
```

FIG. 2

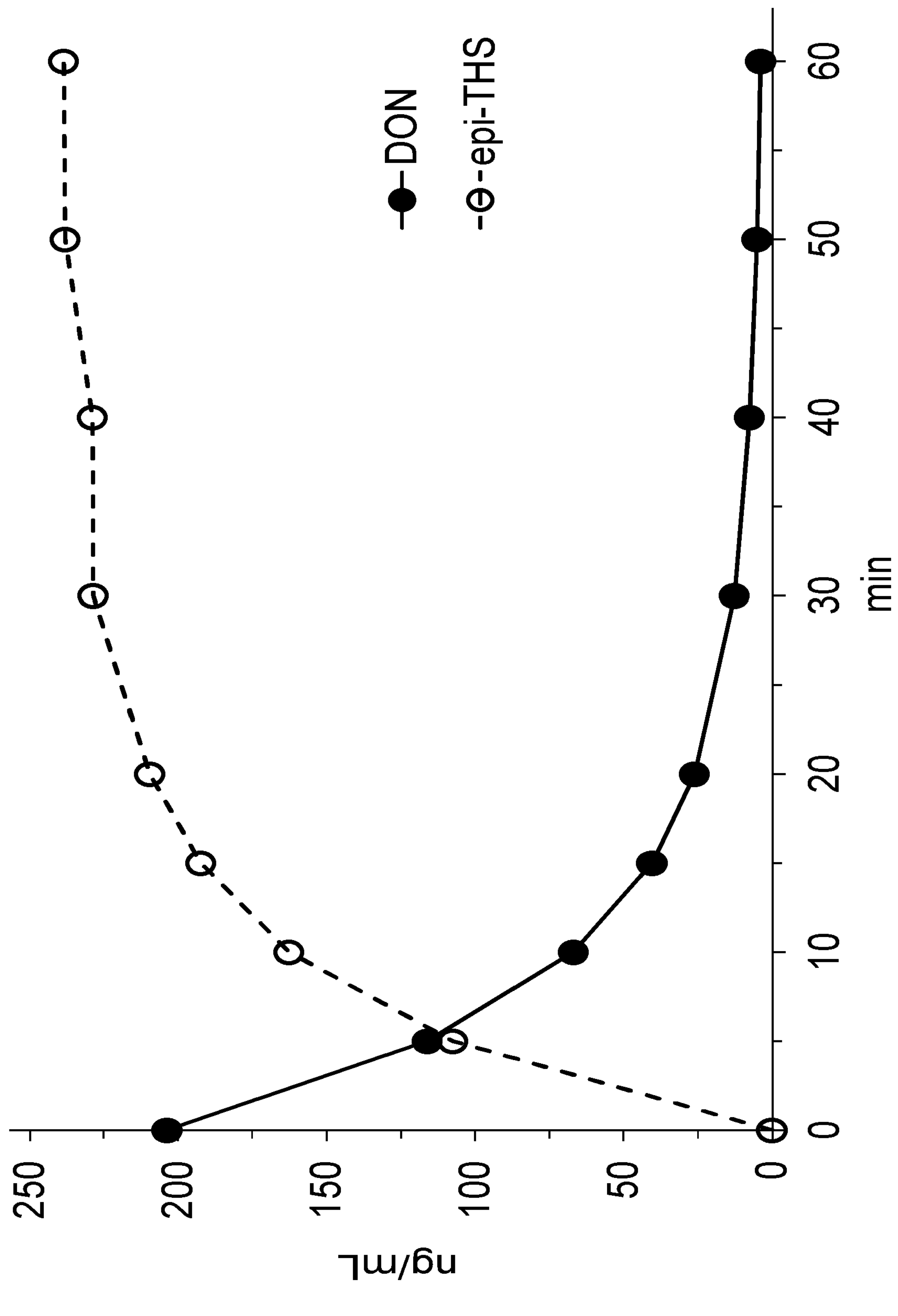

FIG. 3

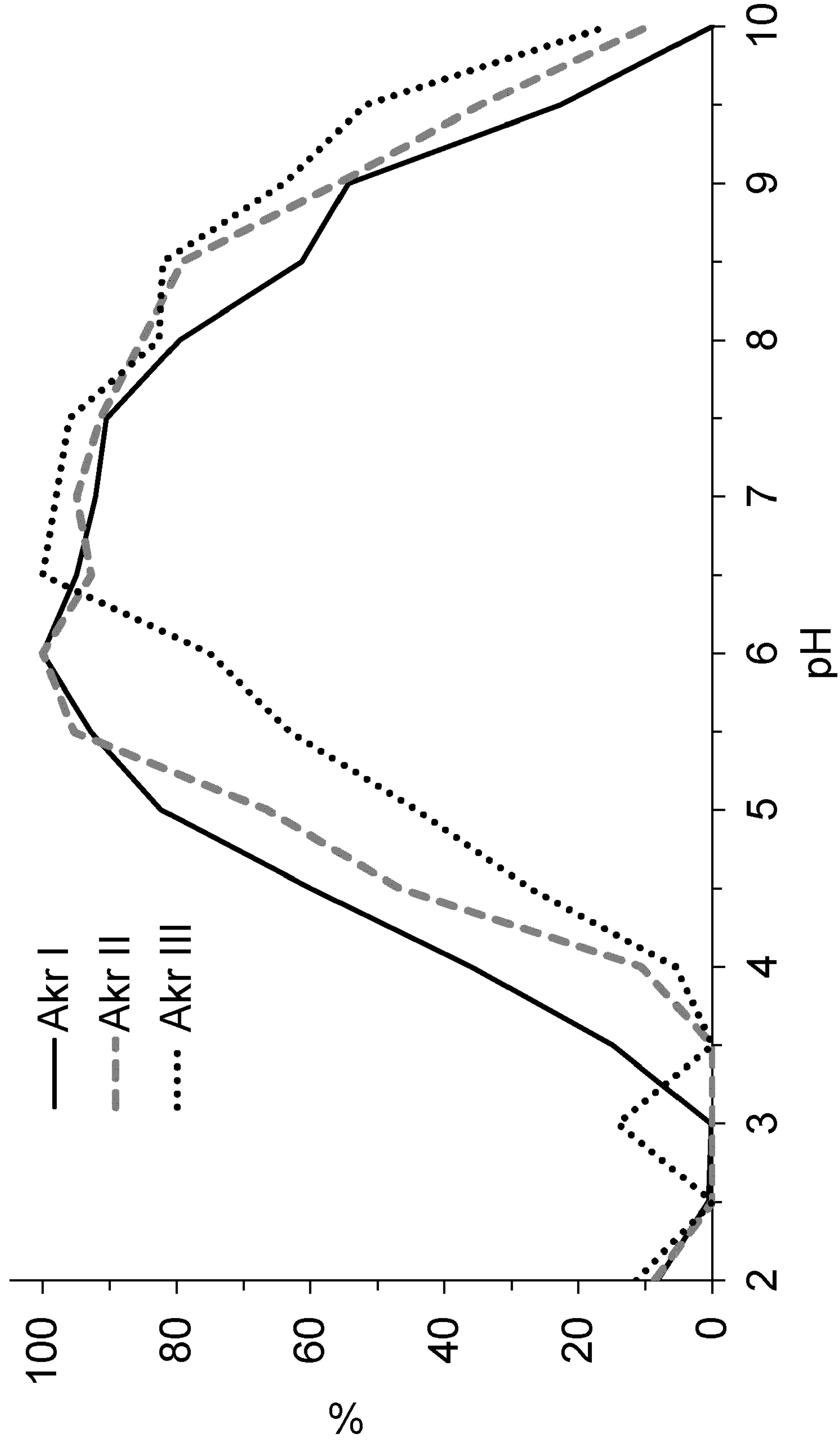

FIG. 4

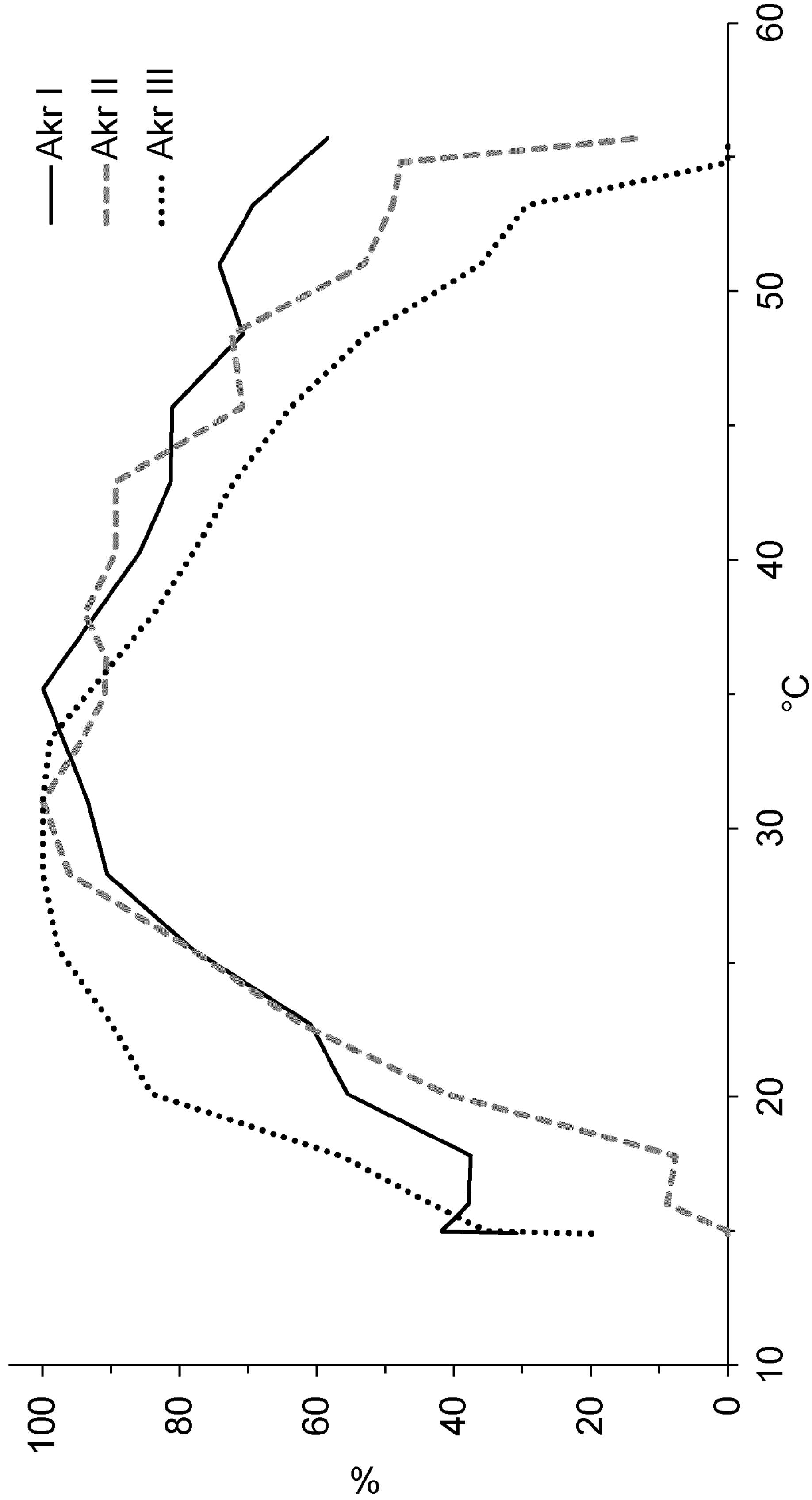

FIG. 5

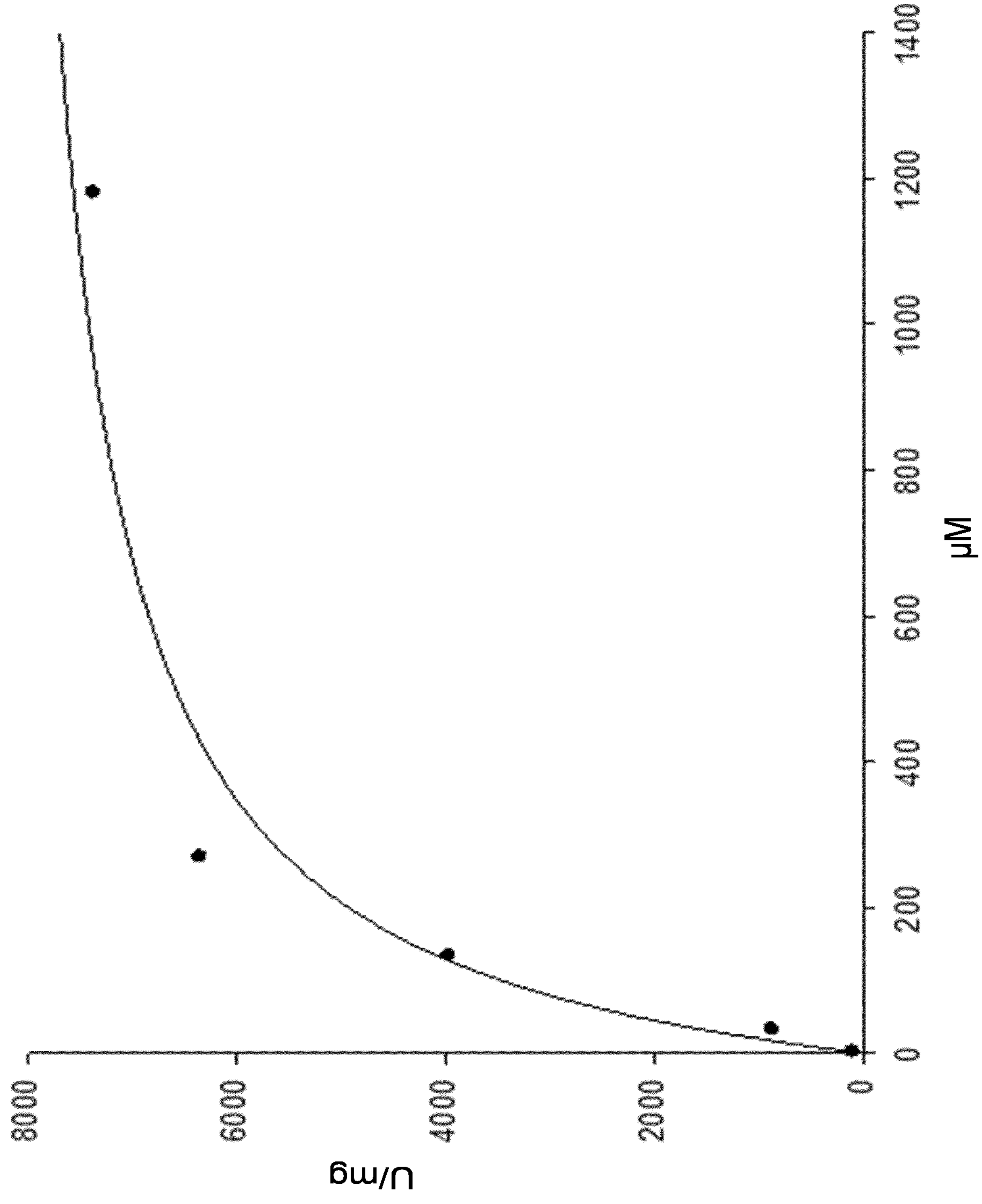

FIG. 6

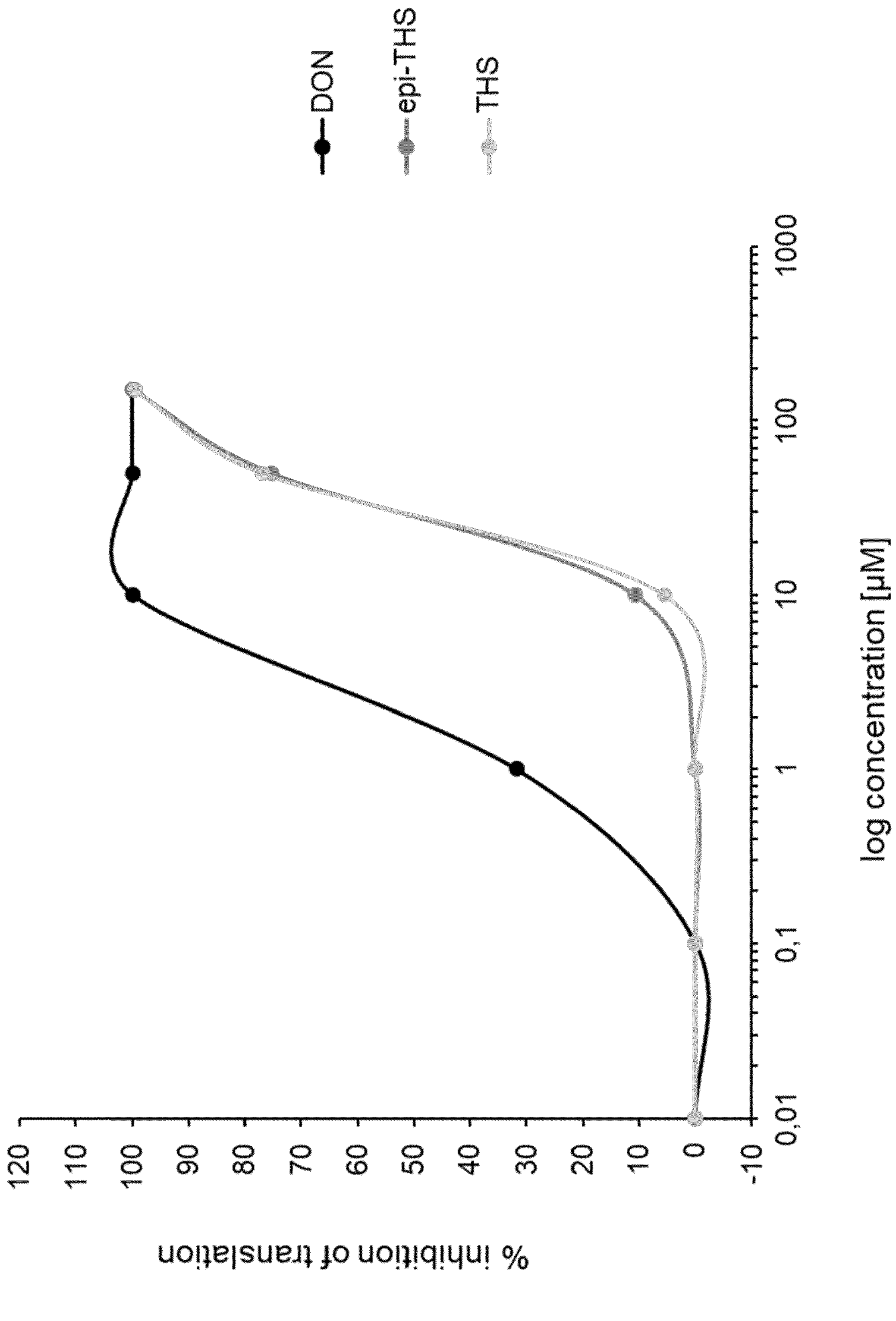
DON
epi-THS
THS
% inhibition of translation
120
110
100
90
80
70
60
50
40
30
20
10
0
-10
0,01
0,1
1
10
100
1000
log concentration [µM]

FIG. 7

a

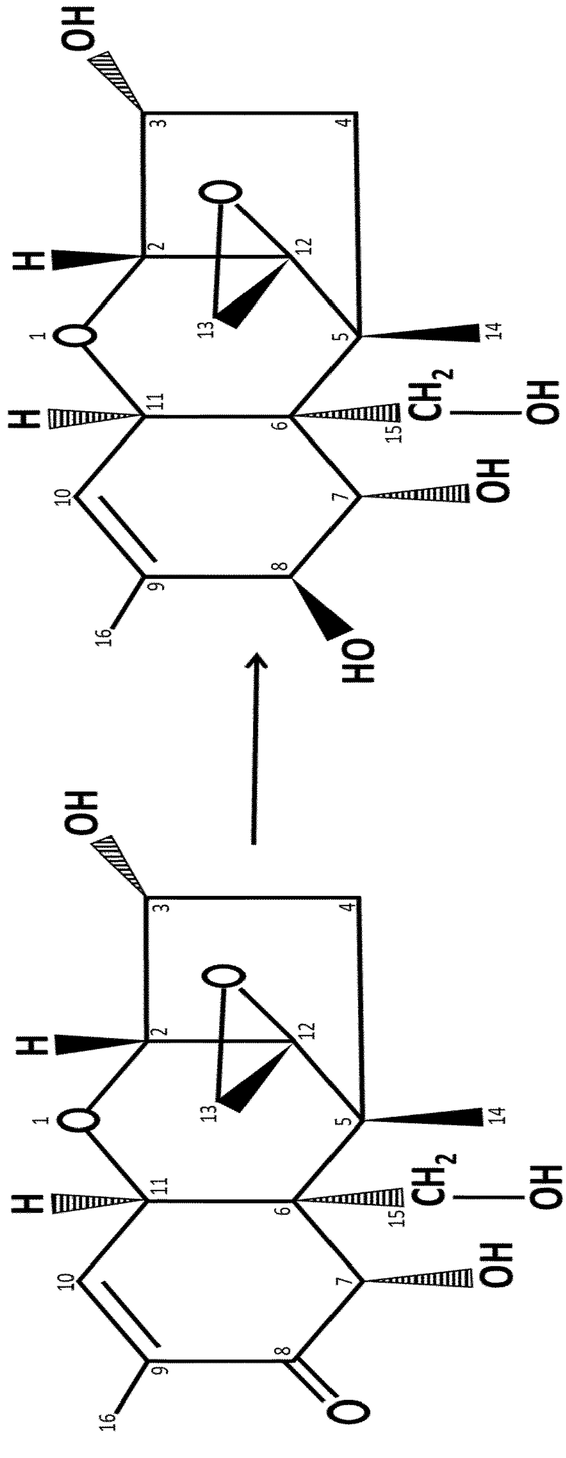

DON

Epi-THS

FIG. 7 (CONT.)

b

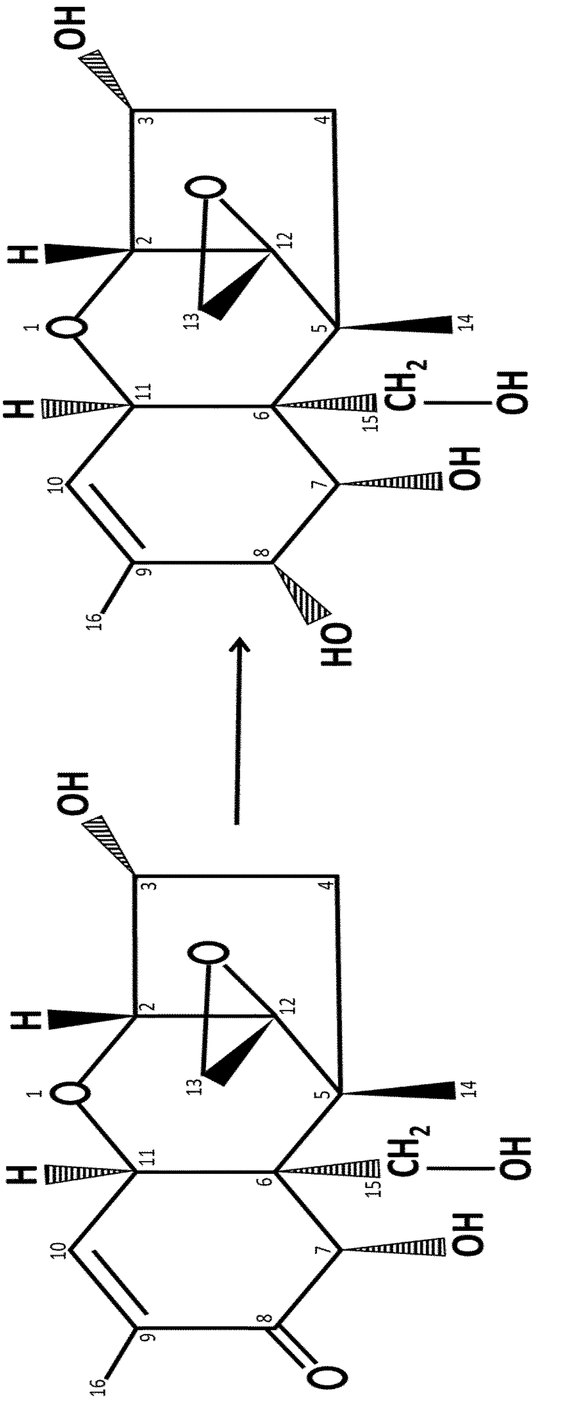

DON

THS

FIG. 7 (CONT.)

C

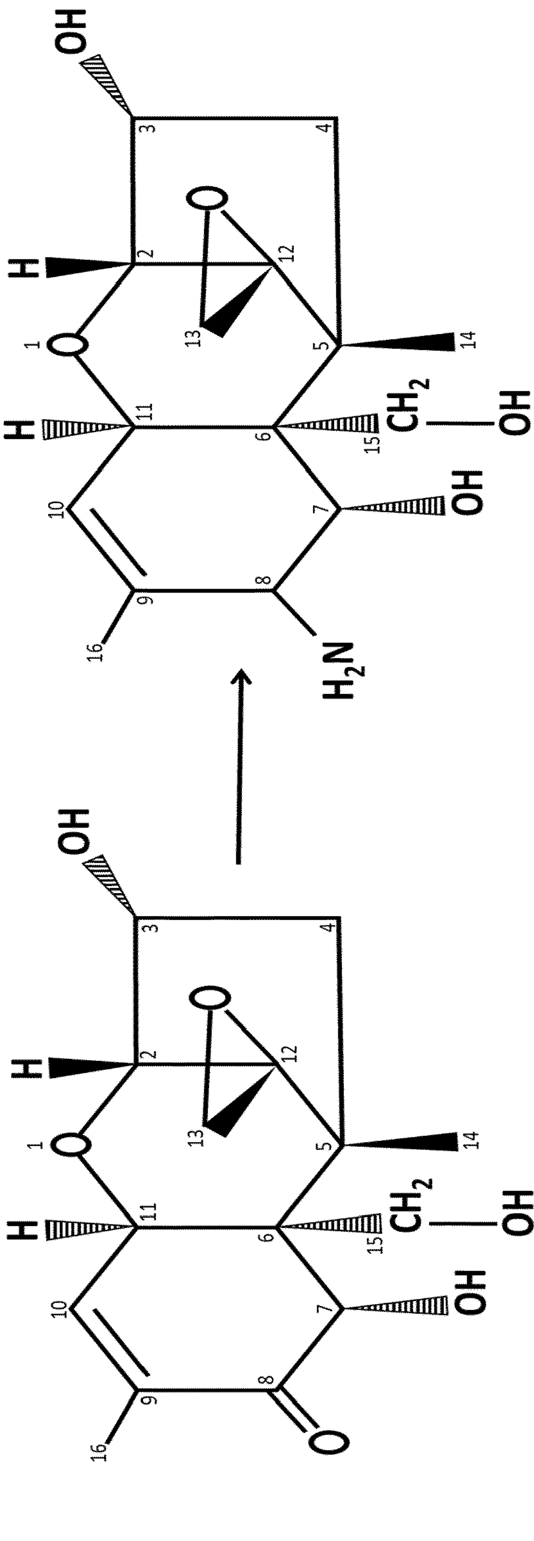

DON

8-amino DON

Europäisches Patentamt
European Patent Office
Office européen des brevets

# EUROPEAN SEARCH REPORT

Application Number
EP 20 19 9475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | DATABASE UniProtKB [Online]<br><br>8 May 2019 (2019-05-08),<br>"SubName: Full=Oxidoreductase {ECO:0000313¦EMBL:GCD91110.1};",<br>XP002802442,<br>retrieved from UniProt<br>Database accession no. A0A401Y8W7<br>* sequence *<br>----- | 1-4 | INV.<br>A23K10/14<br>A23K10/30<br>A23L5/20<br>A23L7/104<br>C12P17/18 |
| X | WO 2012/142302 A2 (CODEXIS INC [US]; GOHEL ANUPAM [SG] ET AL.)<br>18 October 2012 (2012-10-18)<br>* sequence 36 *<br>----- | 1-4 | |
| X | WO 2015/078258 A1 (ASYMCHEM LAB TIANJIN CO LTD [CN] ET AL.) 4 June 2015 (2015-06-04)<br>* sequence 5 *<br>----- | 1-4 | |
| A | WO 02/055522 A2 (ENPHARMA L P [BM]; KRANTIS ANTHONY [CA]; DURST TONY [CA])<br>18 July 2002 (2002-07-18)<br>* page 45; figure 7b *<br>----- | 1 | TECHNICAL FIELDS SEARCHED (IPC)<br><br>C12P<br>C11C<br>A23K<br>A23L |
| A | KONENKO G P ET AL: "3,7,8,15-Tetrahydroxy-12,13-epoxytrichothec-9-en in a culture of Fusarium graminearum",<br>CHEMISTRY OF NATURAL COMPOUNDS,<br>vol. 26, no. 2, 1 March 1990 (1990-03-01),<br>pages 219-220, XP002802443,<br>ISSN: 1573-8388, DOI: 10.1007/BF00607551<br>* the whole document *<br>-----<br>-/-- | 1 | |

2

~~The present search report has been drawn up for all claims~~

| Place of search | Date of completion of the search | Examiner |
|---|---|---|
| The Hague | 22 March 2021 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 20 19 9475

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | FRUHMANN PHILIPP ET AL: "Stereoselective Luche Reduction of Deoxynivalenol and Three of Its Acetylated Derivatives at C8", TOXINS, vol. 6, no. 1, 1 January 2014 (2014-01-01), pages 325-336, XP055787497, CH ISSN: 2072-6651, DOI: 10.3390/toxins6010325 * the whole document * ----- | 1 | |
| A | WO 2006/006082 A2 (ENPHARMA L P; DURST TONY [CA]; KRANTIS ANTHONY [CA]) 19 January 2006 (2006-01-19) * pages 20,22,31 * ----- | 1 | |
| A | MAEDA KAZUYUKI ET AL: "Hydroxylations of trichothecene rings in the biosynthesis of Fusarium trichothecenes: evolution of alternative pathways in the nivalenol chemotype : Hydroxylation steps and genes in the nivalenol pathway", ENVIRONMENTAL MICROBIOLOGY, vol. 18, no. 11, 1 November 2016 (2016-11-01), pages 3798-3811, XP055787487, GB ISSN: 1462-2912, DOI: 10.1111/1462-2920.13338 ----- | 1 | TECHNICAL FIELDS SEARCHED (IPC) |
| A | WO 2016/154640 A1 (ERBER AG [AT]; BINDER EVA-MARIA [AT] ET AL.) 6 October 2016 (2016-10-06) ----- | 1 | |

2

~~The present search report has been drawn up for all claims~~

| Place of search | Date of completion of the search | Examiner |
|---|---|---|
| The Hague | 22 March 2021 | Smalt, Rolf |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

Application Number
EP 20 19 9475

## CLAIMS INCURRING FEES

The present European patent application comprised at the time of filing claims for which payment was due.

☐ Only part of the claims have been paid within the prescribed time limit. The present European search report has been drawn up for those claims for which no payment was due and for those claims for which claims fees have been paid, namely claim(s):

☐ No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-6, 8, 10-12(completely); 13-15(partially)

☐ The present supplementary European search report has been drawn up for those parts of the European patent application which relate to the invention first mentioned in the claims (Rule 164 (1) EPC).

Europäisches Patentamt
European Patent Office
Office européen des brevets

# LACK OF UNITY OF INVENTION SHEET B

Application Number
EP 20 19 9475

The Search Division considers that the present European patent application does not comply with the requirements of unity of invention and relates to several inventions or groups of inventions, namely:

1. claims: 1-6, 8, 10-12(completely); 13-15(partially)

   Enzymes capable of modifying the C-8 atom of a type B trichothecene with the degrees of identity to the respective SEQ.ID.NO's 1-5 as indicated in claim 1, and their uses in processes wherein type B trichothecenes are converted to their 8-hydroxy- or 8-amino-equivalent.

---

2. claims: 7, 9(completely); 13-15(partially)

   Type B trichothecene equivalents having a hydroxy- or amino-functional group at their C-8 position in stead of the keto group, as listed in claims 7 and 9.
   Claims 13-15 have formally been grouped partially with this invention, because they refer to compounds and intermediates of the preceding claims. It is however noted that use in the context described in claim 13-15 of the compounds and intermediates of claims 7 and 9 does not make much practical sense, and must likely be considered as lacking enablement for the indicated purpose.

---

## ANNEX TO THE EUROPEAN SEARCH REPORT ON EUROPEAN PATENT APPLICATION NO. EP 20 19 9475

This annex lists the patent family members relating to the patent documents cited in the above-mentioned European search report. The members are as contained in the European Patent Office EDP file on
The European Patent Office is in no way liable for these particulars which are merely given for the purpose of information.

22-03-2021

| Patent document cited in search report | Publication date | Patent family member(s) | Publication date |
|---|---|---|---|
| WO 2012142302 A2 | 18-10-2012 | DK 2697662 T3 | 30-07-2018 |
| | | EP 2697662 A2 | 19-02-2014 |
| | | HU E038800 T2 | 28-11-2018 |
| | | US 2014199735 A1 | 17-07-2014 |
| | | US 2015329835 A1 | 19-11-2015 |
| | | US 2016244792 A1 | 25-08-2016 |
| | | US 2017159028 A1 | 08-06-2017 |
| | | WO 2012142302 A2 | 18-10-2012 |
| WO 2015078258 A1 | 04-06-2015 | CN 104328093 A | 04-02-2015 |
| | | EP 3075856 A1 | 05-10-2016 |
| | | JP 2016537991 A | 08-12-2016 |
| | | KR 20160089492 A | 27-07-2016 |
| | | US 2017002338 A1 | 05-01-2017 |
| | | WO 2015078258 A1 | 04-06-2015 |
| WO 02055522 A2 | 18-07-2002 | AT 321051 T | 15-04-2006 |
| | | AU 2002224706 B2 | 30-09-2004 |
| | | BR 0206406 A | 10-02-2004 |
| | | CA 2433066 A1 | 18-07-2002 |
| | | CN 1610684 A | 27-04-2005 |
| | | CZ 20031906 A3 | 12-11-2003 |
| | | EP 1366052 A2 | 03-12-2003 |
| | | HU 0401004 A2 | 30-08-2004 |
| | | JP 2004520352 A | 08-07-2004 |
| | | MX PA03006174 A | 12-12-2003 |
| | | NZ 526867 A | 25-11-2005 |
| | | PL 365257 A1 | 27-12-2004 |
| | | WO 02055522 A2 | 18-07-2002 |
| WO 2006006082 A2 | 19-01-2006 | NONE | |
| WO 2016154640 A1 | 06-10-2016 | AR 104043 A1 | 21-06-2017 |
| | | AU 2015388744 A1 | 01-02-2018 |
| | | BR 112017018606 A2 | 17-04-2018 |
| | | CA 2979288 A1 | 06-10-2016 |
| | | CN 107109375 A | 29-08-2017 |
| | | EA 201791911 A1 | 28-02-2018 |
| | | EP 3273802 A1 | 31-01-2018 |
| | | JP 6646680 B2 | 14-02-2020 |
| | | JP 2018509173 A | 05-04-2018 |
| | | SG 11201707010W A | 30-10-2017 |
| | | UA 119813 C2 | 12-08-2019 |
| | | US 2018080011 A1 | 22-03-2018 |
| | | US 2020010812 A1 | 09-01-2020 |
| | | UY 36590 A | 30-09-2016 |

EPO FORM P0459

For more details about this annex : see Official Journal of the European Patent Office, No. 12/82

## ANNEX TO THE EUROPEAN SEARCH REPORT ON EUROPEAN PATENT APPLICATION NO.

EP 20 19 9475

This annex lists the patent family members relating to the patent documents cited in the above-mentioned European search report. The members are as contained in the European Patent Office EDP file on
The European Patent Office is in no way liable for these particulars which are merely given for the purpose of information.

22-03-2021

| Patent document cited in search report | Publication date | Patent family member(s) | Publication date |
|---|---|---|---|
| | | WO 2016154640 A1 | 06-10-2016 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 1042449 A1
- US 20120263827 A1
- WO 2016154640 A1
- CN 107916266 A
- US 2020071638 A
- US 7410800 B2


### Non-patent literature cited in the description


- **SCHATZMAYR ; STREIT.** *World Mycotoxin Journal,* 2013, vol. 6 (3), 213-222
- *CHEMICAL ABSTRACTS,* 51481-10-8
- *CHEMICAL ABSTRACTS,* 21259-20-1
- *CHEMICAL ABSTRACTS,* 26934-87-2
- *CHEMICAL ABSTRACTS,* 23282-20-4
- *CHEMICAL ABSTRACTS,* 23255-69-8
- *CHEMICAL ABSTRACTS,* 2623-22-5
- *CHEMICAL ABSTRACTS,* 2270-40-8
- *CHEMICAL ABSTRACTS,* 2198-93-8
- *CHEMICAL ABSTRACTS,* 3148-09-2
- *CHEMICAL ABSTRACTS,* 4643-58-7
- *CHEMICAL ABSTRACTS,* 91423-90-4
- *CHEMICAL ABSTRACTS,* 344781-02-8
- *CHEMICAL ABSTRACTS,* 38818-51-8
- *CHEMICAL ABSTRACTS,* 34114-99-3
- *CHEMICAL ABSTRACTS,* 74833-39-9
- *CHEMICAL ABSTRACTS,* 36519-25-2
- *CHEMICAL ABSTRACTS,* 65041-92-1
- *CHEMICAL ABSTRACTS,* 101401-89-2
- *CHEMICAL ABSTRACTS,* 109890-37-1
- *CHEMICAL ABSTRACTS,* 90044-33-0
- *CHEMICAL ABSTRACTS,* 18374-83-9
- *CHEMICAL ABSTRACTS,* 51481-10-8, 50722-38-8, 88337-96-6, 23282-20-4, 23255-69-8
- *EFSA Journal,* 2004, vol. 73, 1-41
- **ITO et al.** *Appl Environ Microbiol,* 2012, vol. 79 (5), 1619-1628
- **HE et al.** *Scientific Reports.,* 2017, vol. 27, 9549
- **MINDNICH ; PENNING.** *Hum Genomics,* 2009, vol. 3 (4), 362-370
- **CARERE et al.** *Microb Biotechnol,* 2017
- Enzyme Nomenclature. Academic Press, 1992
- *Eur. J. Biochem.,* 1994, vol. 223, 1-5
- *Eur. J. Biochem.,* 1995, vol. 232, 1-6
- *Eur. J. Biochem.,* 1996, vol. 237, 1-5
- *Eur. J. Biochem.,* 1997, vol. 250, 1-6
- *Eur. J. Biochem.,* 1999, vol. 264, 610-650
- **NEEDLEMAN ; WUNSCH.** *J. Mol. Biol.,* 1970, vol. 48, 443-453
- **RICE et al.** EMBOSS: The European Molecular Biology Open Software Suite. *Trends Genet.,* 2000, vol. 16, 276-277
- **OSTLING ; VIRTAMA.** *Acta Physiologica Scandinavica,* 1946, vol. 11 (4), 289-293
- **ALTSCHUL et al.** *J Mol Biol,* 1990, vol. 215 (3), 403-410
- **CASSIMJEE et al.** *Chem. Commun.,* 2010, vol. 46, 5569-5571
- **KOSZELEWSKI et al.** *Chem. Int. Ed.,* 2008, vol. 47, 9337-9340
- **SHIN ; KIM.** *Biotechnol. Bioeng.,* 1999, vol. 65, 206211

SEQUENCE LISTING

<110>  Erber Aktiengesellschaft

<120>  MEANS AND METHODS TO DETOXIFY MYCOTOXINS

<130>  ERB17122EP

<160>  5

<170>  PatentIn version 3.5

<210>  1
<211>  312
<212>  PRT
<213>  Artificial Sequence

<220>
<223>  ArkI

<400>  1

```
Met Arg Thr Thr Glu Ile Ser Gln Gly Leu Lys Val Ser Asn Leu Cys
1               5                   10                  15

Ile Gly Ala Gly Pro Leu Gly Gly Met Pro Thr Ala Phe Gly Tyr Asp
            20                  25                  30

Val Ser Glu Glu Arg Gly Ile Ala Thr Val Gln Ala Val Phe Asp Ser
        35                  40                  45

Pro Ile Asn Phe Met Asp Thr Ser Ser Glu Tyr Gly Asp Ser Glu Val
    50                  55                  60

Arg Ile Gly Lys Val Ile Arg Glu Gly Arg Met Pro Lys Gly Phe Val
65                  70                  75                  80

Ile Ala Thr Lys Ala Asp Ala Glu Gly Asp Gly Thr Ala Arg Arg Gly
                85                  90                  95

Asp Phe Ser Ala Lys Gln Val Arg Ala Ser Phe Glu Leu Ser Leu Glu
            100                 105                 110

Arg Leu Gly Leu Thr His Ile Asp Val Tyr Phe Leu His Asp Pro Glu
        115                 120                 125

Lys Phe Pro Phe Glu His Val Met Ala Arg Gly Gly Ala Met Glu Glu
    130                 135                 140

Leu Leu Arg Leu Lys Asp Glu Gly Leu Val Gly Leu Val Gly Val Ala
145                 150                 155                 160

Gly Gly Asp Val Val Glu Met Ala Arg Tyr Val Asp Thr Gly Asn Leu
```

165 170 175

Asp Ile Leu Leu Asn Tyr Gly Arg Tyr Thr Leu Leu Asp Arg Ser Ala
180 185 190

Asp Ser Leu Ile Asp Lys Ala Ser Asp Ala Gly Met Ala Phe Leu Asn
195 200 205

Ala Gly Pro Tyr Ala Ser Gly Leu Leu Ala Ala Pro Arg Gly Gly Ser
210 215 220

Ala Trp Tyr Gln Tyr Ala Pro Pro Asn Ala Asp Ala Leu Gln Ala Val
225 230 235 240

Asp Gln Leu His Glu Leu Cys Glu Ser Phe Asp Val Ser Leu Arg Ser
245 250 255

Val Ala Leu Gln Phe Ser Met Arg Asp Pro Arg Ile Thr Ser Thr Val
260 265 270

Val Gly Leu Ser Arg Pro Glu Arg Val Arg Ala Leu Leu Asp Asp Ala
275 280 285

Ala Ala Glu Ile Pro Ala Glu Leu Trp Asp Gln Leu Pro Ala Pro Leu
290 295 300

Asn Leu Pro Trp Asn Ser Leu Ala
305 310

<210> 2
<211> 312
<212> PRT
<213> Artificial Sequence

<220>
<223> ArkII

<400> 2

Met Arg Thr Thr Glu Ile Ala Gln Gly Leu Lys Val Ser Asn Leu Cys
1 5 10 15

Ile Gly Ala Gly Pro Leu Gly Gly Met Pro Thr Ala Phe Gly Tyr Asp
20 25 30

Val Ser Glu Glu Arg Gly Ile Ala Thr Val Gln Ala Val Phe Asp Ser
35 40 45

Pro Ile Asn Phe Met Asp Thr Ser Ser Glu Tyr Gly Gly Ser Glu Ala
50 55 60

```
Arg Ile Gly Lys Val Ile Arg Glu Gly Leu Met Pro Lys Gly Phe Val
65                  70                  75                  80

Ile Ser Ser Lys Ala Asp Ala Glu Gly Glu Gly Val Gly Arg Arg Gly
                85                  90                  95

Asp Phe Ser Ala Lys Gln Val Arg Ala Ser Phe Glu Leu Ser Leu Glu
            100                 105                 110

Arg Leu Gly Leu Thr His Leu Asp Val Tyr Phe Leu His Asp Pro Glu
        115                 120                 125

Lys Phe Pro Phe Glu Gln Val Met Ala Arg Gly Gly Ala Met Glu Glu
    130                 135                 140

Leu Leu Arg Leu Lys Asp Glu Gly Leu Val Gly Leu Val Gly Val Ala
145                 150                 155                 160

Gly Gly Asp Val Leu Glu Met Ala Arg Tyr Val Asp Thr Gly Ser Leu
                165                 170                 175

Asp Ile Leu Leu Asn Tyr Gly Arg Tyr Thr Leu Leu Asp Arg Ser Ala
            180                 185                 190

Asp Ser Leu Ile Thr Lys Val Ser Asp Ala Gly Met Ala Phe Leu Asn
        195                 200                 205

Ala Gly Pro Tyr Ala Ser Gly Leu Leu Ala Ala Pro Arg Gly Gly Ser
    210                 215                 220

Ala Trp Tyr Gln Tyr Ala Pro Pro Asp Ala Thr Ala Leu Gln Ala Val
225                 230                 235                 240

Asp Gln Leu His Glu Leu Cys Glu Ser Phe Asp Val Ser Leu Arg Ser
                245                 250                 255

Val Ala Leu Gln Phe Ser Met Arg Asp Pro Arg Ile Thr Ser Thr Val
            260                 265                 270

Val Gly Leu Ser Arg Pro Glu Arg Val Arg Ala Leu Leu Asp Asp Glu
        275                 280                 285

Ala Ala Glu Ile Pro Ala Glu Leu Trp Glu Gln Leu Pro Ala Pro Leu
    290                 295                 300

Asn Gly Asp Ile Val Leu Pro Gly
```

305 310

<210> 3
<211> 312
<212> PRT
<213> Artificial Sequence

<220>
<223> ArkIII

<400> 3

Met Arg Thr Thr Glu Ile Ala Gln Gly Leu Lys Val Ser Asn Leu Cys
1 5 10 15

Ile Gly Ala Gly Pro Leu Gly Gly Met Pro Thr Ala Phe Gly Tyr Asp
20 25 30

Val Ser Glu Glu Arg Gly Ile Ala Thr Val Gln Ala Val Phe Asp Ser
35 40 45

Pro Ile Asn Phe Met Asp Thr Ser Ser Glu Tyr Gly Gly Ser Glu Ala
50 55 60

Arg Ile Gly Lys Val Ile Arg Glu Gly Arg Met Pro Lys Gly Phe Val
65 70 75 80

Ile Ser Ser Lys Ala Asp Ala Glu Gly Glu Gly Val Gly Arg Arg Gly
85 90 95

Asp Phe Ser Ala Lys Gln Val Arg Ala Ser Phe Glu Leu Ser Leu Glu
100 105 110

Arg Leu Gly Leu Thr His Leu Asp Val Tyr Phe Leu His Asp Pro Glu
115 120 125

Lys Phe Pro Phe Glu Gln Val Met Ala Arg Gly Gly Ala Met Glu Glu
130 135 140

Leu Leu Arg Leu Lys Asp Glu Gly Leu Val Gly Leu Val Gly Val Ala
145 150 155 160

Gly Gly Asp Val Leu Glu Met Ala Arg Tyr Val Asp Thr Gly Ser Leu
165 170 175

Asp Ile Leu Leu Asn Tyr Gly Arg Tyr Thr Ile Leu Asp Arg Ser Ala
180 185 190

Asp Ser Leu Ile Thr Lys Val Ser Asp Ala Gly Met Ala Phe Leu Asn
195 200 205

```
Ala Gly Pro Tyr Ala Ser Gly Leu Leu Ala Ala Pro Arg Gly Gly Ser
    210                 215                 220

Ala Trp Tyr Gln Tyr Ala Pro Pro Asp Ala Thr Ala Leu Gln Ala Val
225                 230                 235                 240

Asp Gln Leu His Glu Leu Cys Glu Ser Phe Asp Val Ser Leu Arg Ser
                245                 250                 255

Val Ala Leu Gln Phe Ser Met Arg Asp Pro Arg Ile Thr Ser Thr Val
            260                 265                 270

Val Gly Leu Ser Arg Pro Glu Arg Val Arg Ala Leu Leu Asp Asp Glu
        275                 280                 285

Ala Ala Glu Ile Pro Ala Glu Leu Trp Glu Gln Leu Pro Ala Pro Leu
    290                 295                 300

Asn Gly Asp Ile Val Leu Pro Arg
305                 310


<210>  4
<211>  252
<212>  PRT
<213>  Artificial Sequence

<220>
<223>  ADH-Lk

<400>  4

Met Thr Asp Arg Leu Lys Gly Lys Val Ala Ile Val Thr Gly Gly Thr
1               5                   10                  15

His Gly Ile Gly Leu Ala Ile Ala Thr Lys Phe Val Thr Glu Gly Ala
            20                  25                  30

Lys Val Val Ile Thr Gly Arg Arg Ala Asp Val Gly Glu Lys Ala Ala
        35                  40                  45

Lys Ser Ile Gly Gly Thr Asp Val Ile Arg Phe Val Gln His Asp Val
    50                  55                  60

Ser Asp Glu Ala Gly Trp Gly Lys Leu Phe Asp Thr Thr Glu Glu Thr
65                  70                  75                  80

Phe Gly Pro Val Thr Thr Leu Val Asn Asn Ala Gly Ile Gly Tyr Arg
                85                  90                  95
```

Lys Ser Val Glu Asp Thr Thr Thr Glu Glu Trp Arg Lys Leu Leu Ser
100 105 110

Val Asn Leu Asp Gly Val Phe Phe Gly Thr Arg Leu Gly Ile Gln Arg
115 120 125

Met Lys Cys Lys Gly Leu Gly Ala Ser Ile Ile Asn Met Ser Ser Ile
130 135 140

Leu Gly Gln Val Gly Asp Pro Ala Thr Gly Ala Tyr Ser Ala Ser Lys
145 150 155 160

Gly Ala Val Arg Ile Met Ser Lys Ser Ala Ala Leu Leu Cys Ala Leu
165 170 175

Lys Asp Tyr Asp Val Arg Val Asn Thr Val His Pro Gly Pro Ile Lys
180 185 190

Thr Pro Leu Met Asp Asp Met Pro Gly Ala Glu Glu Met Met Ser Gln
195 200 205

Arg Thr Lys Thr Pro Met Gly His Ile Gly Glu Pro Asn Asp Ile Ala
210 215 220

Trp Val Cys Val Tyr Leu Ala Ser Asp Glu Ser Lys Phe Ala Thr Gly
225 230 235 240

Ala Glu Phe Val Val Asp Gly Gly Tyr Thr Ala Gln
245 250

<210> 5
<211> 333
<212> PRT
<213> Artificial Sequence

<220>
<223> TAM-Ac

<400> 5

Met Ala Phe Ser Ala Asp Thr Pro Glu Ile Val Tyr Thr His Asp Thr
1 5 10 15

Gly Leu Asp Tyr Ile Thr Tyr Ser Asp Tyr Glu Leu Asp Pro Ala Asn
20 25 30

Pro Leu Ala Gly Gly Ala Ala Trp Ile Glu Gly Ala Phe Val Pro Pro
35 40 45

```
Ser Glu Ala Arg Ile Ser Ile Phe Asp Gln Gly Phe Tyr Thr Ser Asp
    50                  55                  60

Ala Thr Tyr Thr Thr Phe His Val Trp Asn Gly Asn Ala Phe Arg Leu
65                  70                  75                  80

Gly Asp His Ile Glu Arg Leu Phe Ser Asn Ala Glu Ser Ile Arg Leu
                85                  90                  95

Ile Pro Pro Leu Thr Gln Asp Glu Val Lys Glu Ile Ala Leu Glu Leu
            100                 105                 110

Val Ala Lys Thr Glu Leu Arg Glu Ala Met Val Thr Val Thr Ile Thr
        115                 120                 125

Arg Gly Tyr Ser Ser Thr Pro Phe Glu Arg Asp Ile Thr Lys His Arg
    130                 135                 140

Pro Gln Val Tyr Met Ser Ala Cys Pro Tyr Gln Trp Ile Val Pro Phe
145                 150                 155                 160

Asp Arg Ile Arg Asp Gly Val His Leu Met Val Ala Gln Ser Val Arg
                165                 170                 175

Arg Thr Pro Arg Ser Ser Ile Asp Pro Gln Val Lys Asn Phe Gln Trp
            180                 185                 190

Gly Asp Leu Ile Arg Ala Ile Gln Glu Thr His Asp Arg Gly Phe Glu
        195                 200                 205

Leu Pro Leu Leu Leu Asp Cys Asp Asn Leu Leu Ala Glu Gly Pro Gly
    210                 215                 220

Phe Asn Val Val Val Ile Lys Asp Gly Val Val Arg Ser Pro Gly Arg
225                 230                 235                 240

Ala Ala Leu Pro Gly Ile Thr Arg Lys Thr Val Leu Glu Ile Ala Glu
                245                 250                 255

Ser Leu Gly His Glu Ala Ile Leu Ala Asp Ile Thr Pro Ala Glu Leu
            260                 265                 270

Tyr Asp Ala Asp Glu Val Leu Gly Cys Ser Thr Gly Gly Gly Val Trp
        275                 280                 285

Pro Phe Val Ser Val Asp Gly Asn Ser Ile Ser Asp Gly Val Pro Gly
    290                 295                 300
```

```
Pro Val Thr Gln Ser Ile Ile Arg Arg Tyr Trp Glu Leu Asn Val Glu
305                 310                 315                 320

Pro Ser Ser Leu Leu Thr Pro Val Gln Tyr Ala Leu Glu
                325                 330
```